(19) **Europäisches Patentamt**
**European Patent Office**
**Office européen des brevets**

(11) **EP 2 668 496 B1**

(12) **FASCICULE DE BREVET EUROPEEN**

(45) Date de publication et mention
de la délivrance du brevet:
**22.04.2015 Bulletin 2015/17**

(51) Int Cl.:
*G01N 33/50* (2006.01)          *G01N 33/554* (2006.01)
*G01N 33/543* (2006.01)

(21) Numéro de dépôt: **12705371.8**

(22) Date de dépôt: **24.01.2012**

(86) Numéro de dépôt international:
**PCT/FR2012/050157**

(87) Numéro de publication internationale:
**WO 2012/101378 (02.08.2012 Gazette 2012/31)**

(54) **PROCEDE DE FABRICATION D'UN SUPPORT D'ANALYSE ET UTILISATION POUR LA DETECTION DE TOXINES**

VERFAHREN ZUR HERSTELLUNG EINES ANALYSESUBSTRATS UND VERWENDUNG DAVON FÜR DEN NACHWEIS VON TOXINEN

METHOD FOR MANUFACTURING AN ANALYSIS SUBSTRATE, AND USE THEREOF FOR DETECTING TOXINS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **25.01.2011 FR 1150586**

(43) Date de publication de la demande:
**04.12.2013 Bulletin 2013/49**

(73) Titulaires:
• **Centre National de la Recherche Scientifique (CNRS)**
  **75794 Paris Cedex 16 (FR)**
• **Institut Pasteur**
  **75015 Paris (FR)**
• **Universidad De Santiago De Compostela**
  **15782 Santiago de Compostela (ES)**

(72) Inventeurs:
• **ARAOZ, Romulo**
  **91190 Gif-Sur-Yvette (FR)**
• **NGHIEM, Hoang-Oanh**
  **75013 Paris (FR)**
• **MOLGO, Jordi**
  **92160 Antony (FR)**
• **BOTANA, Luis**
  **27002 Lugo (ES)**
• **VILARINO, Natalia**
  **27002 Lugo (ES)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
**EP-A2- 1 376 124**

• **FONFRIA E S ET AL: "Detection of 13,19-didesmethyl C spirolide by fluorescence polarization using Torpedo electrocyte membranes", ANALYTICAL BIOCHEMISTRY, ACADEMIC PRESS INC, NEW YORK, vol. 403, no. 1-2, 1 août 2010 (2010-08-01), pages 102-107, XP027118760, ISSN: 0003-2697 [extrait le 2010-04-09]**
• **VILARIÑO NATALIA ET AL: "Detection of gymnodimine-A and 13-desmethyl C spirolide phycotoxins by fluorescence polarization.", ANALYTICAL CHEMISTRY 1 APR 2009 LNKD-PUBMED:19278248, vol. 81, no. 7, 1 avril 2009 (2009-04-01), pages 2708-2714, XP002653394, ISSN: 1520-6882 cité dans la demande**
• **ARAOZ R ET AL: "A non-radioactive ligand-binding assay for detection of cyanobacterial anatoxins using Torpedo electrocyte membranes", TOXICON, ELMSFORD, NY, US, vol. 52, no. 1, 1 juillet 2008 (2008-07-01), pages 163-174, XP023315844, ISSN: 0041-0101, DOI: DOI:10.1016/J.TOXICON.2008.05.001 [extrait le 2008-05-29] cité dans la demande**

- RÃ MULO ARÃ OZ ET AL: "Ligand-binding assays for cyanobacterial neurotoxins targeting cholinergic receptors", ANALYTICAL AND BIOANALYTICAL CHEMISTRY, SPRINGER, BERLIN, DE, vol. 397, no. 5, 19 mars 2010 (2010-03-19) , pages 1695-1704, XP019839133, ISSN: 1618-2650

**Description**

**Domaine technique**

La présente invention se rapporte à un procédé de fabrication d'un dispositif d'analyse comprenant des fragments de membranes de Torpilles immobilisés à sa surface, au dispositif d'analyse obtenu et à l'utilisation dudit dispositif pour la détection et caractérisation de molécules, en particulier ligands compétitifs des récepteurs nicotiniques de l'acétylcholine.

La présente invention trouve notamment des applications dans le domaine de la surveillance alimentaire de fruits de mer, dans le domaine de la surveillance des réserves d'eau douce, dans le domaine de la recherche médicale, dans le domaine de l'analyse biologique et de la caractérisation de molécules.

Dans la description ci-dessous, les références entre crochets **([ ])** renvoient à la liste des références présentée à la fin du texte.

**Etat de la technique**

Selon des directives européennes récentes DIRECTIVE 2010/63/UE **[1],** le «Test souris», qui était et le seul test officiel utilisé pour la surveillance de toxines marines, doit être remplacé par des méthodes fonctionnelles préalablement validées. Particulièrement en France, le « Test souris » a été remplacé par des méthodes physico-chimiques pour la surveillance sanitaire de fruits de mer à partir de l'année 2010 (Clôture des Assises de la conchyliculture (15/10/2010). Discours de Bruno Le Maire, Ministre de l'Alimentation, de l'Agriculture et de la Pêche)(http://agriculture.gouv.fr/cloture-des-assises-de-la). **[2].**

Les efflorescences de dinoflagellés marins lorsqu'elles sont dominées par une espèce toxique représentent un danger pour l'écosystème marin, les activités commerciales et la santé publique.

En effet, les phycotoxines des dinoflagellés peuvent être accumulées par des mollusques et des poissons et par transport vectoriel peuvent atteindre l'homme (Fleming LE, Broad K, Clement A, Dewailly E, Elmir S, Knap A, Pomponi SA, Smith S, Gabriele HS, Walsh P (2006) Oceans and human health: Emerging public health risks in the marine environment. Mar. Pollut. Bull., 53: 545-560 **[3]**, Molgó J, Girard E, Benoit, E. (2007) Cyclic imines: an insight into this emerging group of bioactive marine toxins. In Phycotoxins: Chemistry and Biochemistry (Botana, L. M., ed) pp. 319-335, Blackwell Publishing Ltd, Iowa. **[4]**). En 2005, le Réseau de Surveillance Phytoplanctonique a détecté grâce au « Test souris » des mollusques contaminés avec des neurotoxines inconnues à partir des échantillons du bassin d'Arcachon. En conséquence l'Etat Français a décrété l'interdiction de la consommation d'huîtres et de coquillages du bassin d'Arcachon et a dû débloquer 2,5 millions d'euros d'aide en faveur de l'Industrie Conchylicole de la Région d'Arcachon, FRANCE. Plus tard, du spirolide A et du 13-desméthyl spirolide-C, toxines produites par le dinoflagellé *Alexandrium ostenfeldii*, ont été détectées dans des huîtres et des moules du bassin d'Arcachon (Amzil Z, Sibat M, Royer F, Masson N, Abadie E. (2007) Report on the first détection of pectenotoxin-2, spirolide-A and their derivatives in French shellfish. Mar. Drugs 5: 168-179.) **[5]**.

Dans le cadre du programme ANR NEUROSPIROIMINE (Programme de Recherche financé par l'Agence Nationale de la Recherche (PCV07-194417-Neurospiroimine)) le mécanisme d'action des imines cycliques de la famille des spirolides et des gymnodimines a été établi (Bourne Y, Radic Z, Aráoz R, Talley TT, Benoit E, Servent D, Taylor P, Molgó J, Marchot P. (2010) Structural déterminants in phycotoxins and AChBP conferring high affinity binding and nicotinic AChR antagonism. Proc. Natl. Acad. Sci. U. S. A. 107: 6076-6081.**[6]**). Les neurotoxines de la famille des spirolides, gymnodimines et pinnatoxines sont des puissants antagonistes des récepteurs nicotiniques de l'acétylcholine (RnACh) de type musculaire et neuronal possédant des affinités dans l'ordre picomolaire et nanomolaire (Bourne Y, Radic Z, Aráoz R, Talley TT, Benoit E, Servent D, Taylor P, Molgó J, Marchot P. (2010) Structural déterminants in phycotoxins and AChBP conferring high affinity binding and nicotinic AChR antagonism. Proc. Natl. Acad. Sci. U. S. A. 107: 6076-6081.**[6]**, Kharrat R, Servent D, Girard E, Ouanounou G, Amar M, Marrouchi R, Benoit E, Molgó J. (2008) The marine phycotoxin gymnodimine targets muscular and neuronal nicotinic acetylcholine receptor subtypes with high affinity. J. Neurochem. 107, 952-963.[7], Aráoz R, Servent D, Molgó J, Iorga BI, Fruchart-Gaillard C, Benoit E, Gu Z, Stivala C, Zakarian A. (2011) Total synthesis of pinnatoxins A and G and revision of the mode of action of pinnatoxin A. J. Am. Chem. Soc. 133, 10499-10511. **[8]**).

Les procédés actuels de détection des toxines dans l'industrie conchylicole, c'est-à-dire de phycotoxines marines sont principalement :

- le « Test souris » qui doit, de part l'évolution des règlements européen, être remplacé par un test n'utilisant pas d'animaux. Actuellement en France, le « Test souris » est seulement utilisé dans le cadre de la vigilance sanitaire de coquillages (Clôture des Assises de la conchyliculture (15/10/2010). Discours de Bruno Le Maire, Ministre de l'Alimentation, de l'Agriculture et de la Pêche)(http://agriculture.gouv.fr/cloture-des-assises-de-la). **[2]**). De plus ce test est difficile à mettre en oeuvre et nécessite des infrastructures *importantes (i.e. animalerie) pour sa mise en oeuvre. En outre*, les professionnels de la conchyliculture remettent en cause de la validité de ce test et contestent les résultats obtenus.

- la chromatographie liquide de haute performance (« High Performance Liquid Chromatography » (HPLC)). Cette méthode est onéreuse nécessitant des appareils importants et couteux et peu sensible.
- HPLC couplée à la Spectrométrie de Masse (LC-MS). Cette méthode est onéreuse nécessitant des appareils importants et couteux et des personnels qualifiés afin de les utiliser. Malgré sa haute sensibilité et spécificité, cette méthode nécessite des toxines standards pour calibrer l'appareil pour leur détection. En plus, la méthode LC-MS est d'utilisation limité dans le cadre de la détection des toxines non-connues. Depuis 2010, la LC-MS est la méthode officielle pour la détection ou la surveillance de phycotoxines marines qui sont sujet à réglementation en France, reléguant ainsi le Test souris au rôle de vigilance sanitaire des fruits de mer (Clôture des Assises de la conchyliculture (15/10/2010). Discours de Bruno Le Maire, Ministre de l'Alimentation, de l'Agriculture et de la Pêche)(http://agriculture.gouv.fr/cloture-des-assises-de-la). **[2])**. Toutefois, la méthode LC-MS ne permet pas et ne peut pas être appliquée pour la détection des nouvelles toxines.

Il existe donc un réel besoin de trouver un procédé et/ou un dispositif simple et peu onéreux, par exemple un test fonctionnel permettant la détection de toxines, en particulier de neurotoxines, par exemple des phycotoxines de la famille des imines cyclisées, par exemple des gymnodimines, spirolides, pinnatoxines, pteriatoxines, prorocentrolides et spiro-prorocentrimine ou de nouvelles toxines, par exemple la pinnamine, agissant sur les RnACh, par exemple pour la surveillance de phytoplancton neurotoxique pour l'industrie conchylicole ou la qualité des eaux de baignade dans les plages pour le tourisme, ainsi que pour la surveillance de fruits de mer contaminés.

De même, la prolifération de cyanobactéries dans les réserves d'eau douce constitue un danger potentiel pour la santé publique. En effet, des espèces de cyanobactéries peuvent produire des neurotoxines, il peut s'agir par exemple des espèces toxiques du genre *Anabaena, Oscillatoria* ou autres produisant de l'anatoxine-a ou homoanatoxine-a (Sivonen K, Jones G. (1999) Cyanobacterial toxins. In Toxic cyanobacteria in water: a guide to their public health consequences, monitoring and management (Chorus, I., ed) pp. 41-111, Bartram, J. E. & F.N. Spon, London. **[9])**. Certes, de nombreux épisodes d'envenimement de chiens ayant bu de l'eau à fort contenu en cyanobactéries produisant de l'anatoxine-a et de l'homoanatoxine-a, puissants agonistes des RnACh, se sont produits en France dans des fleuves-comme La Loue (2003) et Le Tarn (2002, 2003 et 2005) (Gugger M, Lenoir S, Berger C, Ledreux A, Druart JC, Humbert JF, Guette C, Bernard C. (2005) First report in a river in France of the benthic cyanobacterium Phormidium favosum producing anatoxin-a associated with dog neurotoxicosis. Toxicon 45, 919-928. **[10]**, Cadel-Six S. Peyraud-Thomas C, Brient L, Tandeau de Marsac N, Rippka R, Mejean A. (2007) Different genotypes of anatoxin-producing cyanobacteria coexist in the Tarn River, France. Applied and Environmental Microbiology 73, 7605-7614. **[11])**.

Les procédés actuels de détection des neurotoxines de cyanobactéries sont principalement :

- HPLC : cette méthode est onéreuse nécessitant des appareils importants et couteux et peu sensible.
- HPLC couplée à la Spectrométrie de Masse (LC-MS). Cette méthode est onéreuse et nécessite des appareils importants et couteux ainsi que des personnels qualifiés afin de les utiliser.

Il existe également dans l'état de la technique des procédés de détection en solution de composés tel que le 13, 19 didesmethyl spirolide C, la gymnodimine A et le 13-desmethyl spirolide C (Fonfria et coll. « détection of 13,19 didesmethyl spirolide C by fluorescense polarization using torpedo electrocyte membranes » Analytical Biochemistry, Vol 403: 1-2, 2010, p 102-107) **[12]**, Vilariño et coll. « detection of gymnodimine-A and 13-desmethyl spirolide C phycotoxins by fluorescense polarization », Analytical Chemistry, Vol 81: 7, p 2708-2714 **[13]**; ou de l'anatoxine-a et de l'homoanatoxine-a de cyanobactéries (Aráoz et coll. « A non radioactive ligand-binding assay for détection of cyanobacterial anatoxins using torpedo electrocytes membranes », Toxicon, Vol 52:1, p 163-174 **[14])**. Toutefois, ces procédés ont des sensibilités de détection faibles et nécessitent pour la détection desdits composés l'utilisation d'appareils importants, tel que des fluoromètres à polarisation, et des détecteurs de chimiluminescence couteux et complexes nécessitant ainsi du personnels qualifiés pour leur mise en oeuvre et utilisation.

ROMULO ARAOZ ET AL [21]: "Ligand-binding assays for cyanobacterial neurotoxins targeting cholinergic receptors", ANALYTICAL AND BIOANALYTICAL CHEMISTRY, SPRINGER, BERLIN, DE, vol. 397, no. 5, mars 2010, pages 1695-1704, décrit la détection / le criblage de ligands de récepteurs nicotiniques de l'acétylcholine à l'aide de membranes de Torpilles en solution.

EP 1 376 124 A2 décrit des procédés de détection de molécules chimiques et biologiques utilisant un support avec une pluralité de membranes biologiques associé a sa surface, sa mise en contact avec un échantillon et la mesure de l'activité de liaison.

Il existe donc un réel besoin de trouver un procédé et/ou un dispositif, en particulier un dispositif simple et peu onéreux, par exemple un test fonctionnel, permettant d'identifier des toxines de cyanobactéries agissant sur les RnACh, par exemple afin de contrôler la qualité des eaux de reserves d'eau douce, la surveillance des suppléments alimentaires à base de cyanobactéries, la qualité des eaux de baignade, permettant de réduire les coûts, le temps de mise en oeuvre et d'améliorer la fiabilité des résultats obtenus.

## Description de l'invention

La présente invention permet de résoudre et de surmonter les obstacles et inconvénients de l'art antérieur précités en fournissant un procédé de fabrication d'un dispositif d'analyse comprenant des fragments de membranes de Torpilles immobilisés à sa surface comprenant les étapes de :

a. Fragmentation de membranes isolées à partir de cellules électrocytes de Torpille,
b. Incorporation des fragments membranaires obtenus à l'étape a) dans une solution de pH compris de 6,5 à 10
c. Fixation desdits fragments membranaires à la surface du dispositif.

Selon l'invention, par cellules électrocytes on entend des cellules musculaires modifiées ayant perdu leur capacité de contraction et qui se sont spécialisées à générer de l'électricité. Les électrocytes peuvent être issues de l'organe électrique de poissons électriques, par exemple des espèces de la famille *Torpedinidae* comme les Torpilles, ou de la famille *Electrophoridae* comme l'anguille électrique (*Electrophorus electricus*).

Selon l'invention, les cellules peuvent être des cellules issues de l'organe électrique de Torpilles. Par exemple des electrocytes de Torpilles choisies dans le groupe comprenant *Torpedo adenensis, Torpedo alexandrinsis* ou Torpille alexandrine, *Torpedo andersoni* ou Torpille de Floride, *Torpedo bauchotae, Torpedo californica* ou Raie électrique du Pacifique, *Torpedo fairchildi, Torpedo fuscomaculata, Torpedo mackayana, Torpedo macneilli, Torpedo marmorata* ou Raie électrique marbrée ou Torpille marbrée, *Torpedo microdiscus, Torpedo nobiliana* ou Raie électrique de l'Atlantique, *Torpedo panthera* ou Torpille panthère, *Torpedo peruana, Torpedo semipelagica, Torpedo sinuspersici, Torpedo suessii, Torpedo tokionis, Torpedo torpedo* ou Torpille commune, *Torpedo tremens*. De préférence les cellules utilisées sont des cellules de *Torpedo marmorate* ou *Torpedo californica.*

L'isolement des membranes peut préalablement comprendre le prélèvement du tissu électrique de Torpille. Cette étape de prélèvement du tissu électrique de Torpille peut être réalisée par tout procédé connu de l'homme du métier. Il peut s'agir, par exemple, du procédé décrit dans le document Morel et coll. (1985)Morel N, Marsal J, Manaranche R, Lazereg S, Mazie JC, Israel M (1985). Large-scale purification of presynaptic plasma membranes from Torpedo marmorata electric organ. J. Cell Biol., 101: 1757-1762. **[15]**. Il peut s'agir d'un procédé comprenant, une fois la Torpille endormie sur une couche de glace, par exemple en déposant le poisson sur ladite couche pendant environ 20 min, deux incisions au niveau des zones latérales du crâne sont pratiqués à l'aide d'un bistouri pour couper les 4 nerfs principaux qui relient chaque organe électrique avec le *lobus electricus* situé en derrière le *cerebellum* du poisson. Les deux organes électriques, une fois prélevés sont trempés dans une solution tampon, comprenant par exemple 280 mM de Chlorure de Sodium (NaCl), 3 mM de chlorure de potassium (KCl), 1,8 mM de chlorure de Magnésium ($MgCl_2$), 3,4 mM de chlorure de calcium ($CaCl_2$), 5 mM de bicarbonate de sodium $NaHCO_3$, 1,2 mM de tampon phosphate, 5,5 mM de glucose, 300 mM d'urée et 100 mM de sucrose.

Le procédé peut comprendre en outre une étape de préparation du tissu électrique de Torpille obtenu, préalablement à l'isolement des membranes.

La préparation peut être réalisée, par exemple par immersion du tissu électrique prélevé dans une solution d'extraction, par exemple un tampon d'extraction de membranes d'électrocytes (TEME) comprenant 50 mM de Tris-HCl (pH 7,5), 3 mM de EDTA, 1 mM d'acide tétraacétique d'éthylène glycol (EGTA) et des inhibiteurs de protéases.

Le tissu peut être ensuite découpé finement, par exemple à une épaisseur de 1 à 5 mm à l'aide d'un bistouri et immergé dans une solution fraichement préparée, par exemple le tampon TEME comprenant 50 mM de Tris-HCl (pH 7,5), 3 mM de EDTA, 1 mM d'acide tétraacétique d'éthylène glycol (EGTA) et des inhibiteurs de protéases.

La solution d'immersion peut être préparée extemporanément, par exemple dans la journée, par exemple 10 heures avant, par exemple 5 heures avant, par exemple 2 heures avant.

L'isolement de membranes à partir des organes électrique prélevés et préparés comme décrit préalablement peut être réalisée par sédimentation, par exemple dans un gradient discontinu de sucrose, par exemple selon le procédé décrit dans le document Hill et coll. (1991)Hill JA, Nghiêm H-O & Changeux J-P (1991). Serine-specific phosphorylation of nicotinic receptor associated 43K protein. Biochemistry, 30, 5579-5585. **[16]** ou Vilariño et coll. (2009) Vilariño N, Fonfria ES, Molgó J, Aráoz R, Botana LM (2009). Detection of Gymnodimine-A and 13-Desmethyl C Spirolide Phycotoxins by Fluorescence Polarization. Analytical Chemistry, 81: 2708-2714. **[13]**. Par exemple un tissue préparé, par exemple tel que décrit dans l'étape de préparation, est homogénéisé dans un tampon, par exemple du TEME tel que défini précédemment à l'aide d'un broyeur à couteaux, puis la fraction membranaire totale est précipitée par centrifugation, par exemple à 20,000 tours par minute et reprise dans un tampon, par exemple du TEME, contenant 10 à 45% de sucrose, de préférence 35% de sucrose. Le gradient discontinu de sucrose peut être réalisé, par exemple avec, par exemple deux solutions de sucrose de 60 à 10% préparées dans le tampon TEME en poids par rapport au poids total de la solution, par exemple de solution à 60 et 20%, à 30 et 50 %, à 35 et 45 % de sucrose. Selon l'invention, une des solutions de sucrose peut comprendre les membranes totales d'électrocytes de Torpille. Par exemple, il peut s'agir de deux solutions de sucrose à 35% et à 45%, la solution à 35% de sucrose contenant les membranes totales d'électrocytes de Torpille. Après ultracentrifugation, les membranes d'électrocytes sédimentent à l'interphase entre les solutions de sucrose à 35% et à 45% d'où elles sont récupérées et lavées par ultracentrifugation, par exemple de 20,000 à 40,000, de préférence à 40,000 tours par minute dans du tampon glycine 5 mM.

Dans un autre mode de réalisation, après ultracentrifugation, par exemple de 20,000 à 40,000, de préférence à 40,000 tours par minute pendant par exemple de 1 à 5 heures, de préférence pendant 3 h, les membranes d'électrocytes sédimentent à l'interphase entre les solutions de sucrose à 35% et à 45% d'où elles sont récupérées et lavées par ultracentrifugation, par exemple de 20,000 à 40,000, de préférence à 40,000 tours par minute pendant par exemple de 1 à 60 minutes, de préférence de pendant 30 min dans du tampon glycine 5 mM.

Selon l'invention les membranes de Torpilles peuvent être des membranes de cellules électrocytes, de préférence sont préparées à partir de cellules électrocytes de Torpilles indiquées précédemment.

Les membranes d'électrocyte de Torpille sont des membranes comprenant des récepteurs nicotiniques de l'acétylcholine (RnACh), en particulier des récepteurs de type musculaire qui sont constitués par deux sous-unités alpha 1, une sous-unité beta 1, une sous-unité gamma et une sous-unité delta ($\alpha 1_2 \beta \gamma \delta$) (Changeux JP (2010). Allosteric receptors: from electric organ to cognition. Annu. Rev. Pharmacol. Toxicol., 50: 1-38 [17]).

Les membranes d'électrocyte de Torpille sont riches en RnACh, représentant de 10 à 60%, de 20 à 60%, de préférence de 20 à 40% de la concentration totale de protéines.

Selon l'invention la fragmentation des membranes d'electrocyte peut être réalisée par fragmentation mécanique, par exemple à l'aide d'un broyeur, par exemple un broyeur en verre, par exemple un broyeur en verre de borosilicate de type Potter-Elvehjem avec piston en Téflon, par fragmentation aux ultrasons, par exemple par sonication. De préférence, le broyeur est un broyeur en verre de borosilicate de type Potter Elvehjem avec un piston en Téflon, par exemple dans du tampon glycine de 1 à 10 mM, de préférence 5 mM glycine. Avantageusement l'utilisation d'un broyeur verre/téflon permet de préserver la fonctionnalité des RnACh.

Selon l'invention la préparation des solutions stock des fragments membranaires peut être réalisée par tout procédé connu de l'homme du métier, il peut s'agir par exemple d'une mise en solution des fragments isolées selon le procédé décrit précédemment, par exemple avec un Potter-Elvehjem, dans une solution comprenant de 1 à 10 mM de glycine, de préférence 5 mM glycine.

Selon l'invention, la concentration en protéine de la solution stock des fragments membranaire d'électrocyte de Torpille peut être par exemple de 0,5 à 10 mg/ml. Selon l'invention, la concentration de protéine totale des membranes de Torpille peut être ajustée par exemple à une concentration de 0,5 à 5, de 1 à 4, de 1,5 à 3,5, de préférence de 2,5 à 3.5 mg/ml de protéine.

Selon l'invention la solution comprenant les fragments membranaires pour le recouvrement (« coating ») des plaques, peut être une solution de pH compris entre 6,5 à 10, cette solution correspond à la solution dans laquelle sont incorporés les fragments membranaires. Il peut s'agir par exemple d'une solution tampon tris salin (TBS) comprenant 150 mM chlorure de sodium, 50 mM Tris-HCl ou Tris, pH 7,5, d'une solution tampon phosphate salin (PBS) comprenant 130 mM chlorure de sodium, 10 mM sodium phosphate, pH 7,0 ou une solution tampon carbonate/bicarbonate comprenant 150 mM chlorure de sodium, 100 mM sodium carbonate, pH 9,5.

Avantageusement, le pH de la solution comprenant les fragments membranaires pour le recouvrement (« coating ») permet de favoriser l'interaction entre le ligand et le RnACh. En outre, le pH de la solution de recouvrement permet avantageusement de stabiliser les fragments membranaires et que les membranes fixées soient biologiquement actives.

Selon l'invention la concentration de protéine totale dans ladite solution de recouvrement de pH compris de 6,5 à 10 peut être comprise entre 5 à 200 $\mu$g/ml.

Selon l'invention, la force ionique de la solution comprenant les fragments membranaires pour le recouvrement (« coating ») des plaques peut être de 0,1 à 0,7, de préférence de 0,16 à 0,42.

Selon l'invention, le calcul de la force ionique de la solution peut être réalisé par toute méthode connue de l'homme du métier. Par exemple, il peut s'agir du résultat obtenu à partir de la formule (I) suivante :

$$I = \frac{1}{2} \sum_i z_i^2 [x_i]$$

(I)

où, z, représente la charge de l'ion i présente dans ladite solution à une concentration molaire [X*i*].

Avantageusement, la force ionique de la solution comprenant les fragments membranaires pour le recouvrement (« coating ») favorise l'interaction entre le ligand et le RnACh. En outre, la force ionique de la solution de recouvrement permet avantageusement de stabiliser les fragments membranaires et que les membranes fixées soient biologiquement actives.

Selon l'invention, la fixation des fragments de membranes peut être réalisée par exemple par interaction non-covalente et non-ionique entre les fragments membranaires et la surface.

Selon l'invention, le procédé de l'invention peut comprendre préalablement à l'étape c) de fixation une étape b') de dilution des fragments membranaires dans une solution.

Selon l'invention la dilution peut être réalisée par exemple dans une solution physiologique, par exemple une solution saline, par exemple d'une solution tampon tris salin (TBS) comprenant 150 mM chlorure de sodium, 50 mM Tris-HCl, pH 7,5, d'une solution tampon phosphate salin (PBS) comprenant 130 mM chlorure de sodium, 10 mM sodium phosphate, pH 7,0 ou une solution tampon carbonate/bicarbonate comprenant 150 mM chlorure de sodium, 100 mM sodium carbonate, pH 9,5.

Selon l'invention, le dispositif peut être tout dispositif connu de l'homme du métier pour la fixation de molécules biologiques, par exemple, il peut s'agir d'un dispositif dont la surface est en plastique. Il peut s'agir, par exemple d'une plaque multipuits, par exemple une plaque de 6, 12, 24, 48, 96, 384 puits, de préférence 96 ou 384 puits, une plaque de 4, 8 ou 12 puits ou des barrettes à 6, 8 ou 12 puits. Il peut s'agir, par exemple, d'une plaque ELISA, une plaque de haute adhérence pour les molécules polaires, par exemple des plaques de type NUNC Polysorp™ (marque de commerce), Medisorp™ (marque de commerce), Maxisorp™ (marque de commerce), ou Multisorp™ (marque de commerce). Il peut s'agir par exemple de plaques à haute adhérence pour des glycoprotéines (la liste de microplaques n'est pas limitative).

De préférence, la plaque est une plaque Maxisorp™ (marque de commerce). Avantageusement la plaque Maxisorp™ (marque de commerce) dispose en plus de groupes hydrophobes, de beaucoup groupes hydrophyles augmentant sa capacité à établir des liaisons d'hydrogène en plus des interactions de type van der Waals (Esser P (2010). Principles in Adsorption to Polystyrene. Technical Bulletin: 06a. Thermo Fisher Scientific Inc. **[18]**).

Selon l'invention, lorsque qu'une plaque multipuits est utilisée, la quantité de protéine des fragments membranaires peut être de 0,5 à 5 μg de protéine par puits, par exemple de 1 à 2 μg de protéine par puits, de 1 à 1,5 μg de protéine par puits.

Selon l'invention, le procédé peut être mis en oeuvre, par exemple avec des solutions stock de membranes d'électrocyte de Torpille.

Selon l'invention, le procédé peut être mis en oeuvre avec des solutions stock préalablement obtenues par exemple, par mise en oeuvre des étapes a) et b) du procédé de l'invention.

Selon l'invention la solution stock peut être toute solution connue de l'homme du métier adaptée à la conservation de fragments de membrane, par exemple une solution physiologique, par exemple du TBS, du PBS, une solution de glycine, par exemple une solution de glycine à une concentration de 1 mM à 10 mM, de 3 à 8 mM, de préférence 5 mM.

Dans ce cas, le procédé de l'invention comprend l'étape c) de fixation des membranes à partir de solutions stocks.

Selon l'invention les solutions stock peuvent être préalablement aliquotées, par exemple il peut s'agir de solutions de volume de 10 μl à 1 ml, par exemple de 100 μl à 500 μl, de préférence de 500 μl.

Selon l'invention la concentration de protéines dans la solution stock peut être comprise entre 0,5 à 5, de 1 à 4, de 1,5 à 3,5 mg/ml, de préférence de 2,5 à 3,5 mg/ml de protéine.

Avantageusement la concentration de protéine permet d'éviter notamment la décongélation/ congélation répétitive des échantillons membranaires et ainsi préserve la fonctionnalité des RnACh.

Avantageusement, la conservation des fragments membranaires d'électrocyte de Torpille en aliquotes permet également d'éviter notamment la décongélation/ congélation répétitive des échantillons membranaires et ainsi préserve la fonctionnalité des RnACh.

Selon l'invention, l'étape c) de fixation peut être réalisée pendant un temps déterminé, par exemple cette étape peut être réalisée pendant au moins 10 minutes, de préférence au moins 60 minutes, par exemple l'étape d) peut être réalisée pendant 1 à 30 heures, par exemple de 1 h à 30 h, de 3h à 24 h, de 6h à 12 h. L'étape c) peut être également réalisée pendant toute une nuit, par exemple pendant de 16 à 18 heures

Avantageusement la durée de l'étape c) permet de stabiliser la fixation des fragments membranaires d'électrocyte de Torpille sur la surface des puits augmentant ainsi la sensibilité de détection du dispositif. Cette stabilisation permet avantageusement un transport desdits dispositifs par voie aérienne ou terrestre par exemple en Europe ou vers d'autres continents sans altération de la sensibilité du procédé de la présente invention et de son dispositif de mise en oeuvre.

Selon l'invention, lorsqu'une solution stock est utilisée dans le procédé de l'invention, il peut comprendre l'étape b') de dilution de la solution stock.

Selon l'invention la dilution de la solution stock peut être de 50 à 500 fois, de préférence de 100 à 300 fois, de manière encore plus préférée de 200-fois à 250-fois.

Selon l'invention la dilution de la solution peut permettre d'obtenir par exemple une concentration de protéines totale dans les fragments de membrane d'électrocyte de Torpille de 5 μg/ml à 200 μg/ml, de préférence de 10 μg/ml à 20 μg/ml de protéine total par puits.

Selon l'invention la dilution de la solution peut permettre également d'obtenir par exemple une concentration de protéines totale dans les fragments de membrane d'électrocyte de Torpille de 1 μg/ml à 200 μg/ml, de préférence de 5 μg/ml à 100 μg/ml de protéine total par puits.

De préférence lorsque le procédé est mis en oeuvre avec des solutions stock de membranes d'électrocyte de Torpille, il peut s'agir de solutions stocks diluées comme indiqué précédemment.

La présente invention à également pour objet un dispositif d'analyse obtenu par le procédé selon l'invention.

La présente invention à également pour objet l'utilisation d'un dispositif d'analyse selon l'invention pour la détection et la quantification de toxines, par exemple des neurotoxines.

Selon l'invention les neurotoxines peuvent être toute neurotoxines connues de l'homme du métier, par exemple, il peut s'agir de neurotoxines agissant, par exemple sur les récepteurs nicotiniques de l'acétylcholine, de neurotoxines produites par du phytoplankton marin comme certains membres du genre *Alexandrium,* par exemple *Alexandrium ostenfeldii*, produisant par exemple du spirolide, des membres du genre *Karenia,* par exemple *Karenia selliformis*, produisant par exemple de la gymnodimine, des phycotoxins de la famille des spiroimines par exemple les pinnatoxines, les ptériatoxines, les prorocentrolides ou le spiro-prorocentrimine susceptibles d'être produites par des différents espèces de phytoplancton (Molgó J, Girard E, Benoit, E. (2007) Cyclic imines: an insight into this emerging group of bioactive marine toxins. In Phycotoxins: Chemistry and Biochemistry (Botana, L. M., ed) pp. 319-335, Blackwell Publishing Ltd, Iowa. [4]. Il peut s'agir aussi de neurotoxines des cyanobactéries, par exemple l'anatoxine-a, l'homoanatoxine-a, produites par exemple par des membres des genres *Anabaena, Aphanizomenon, Cylindrospermum, Microcystis, Oscillatoria, Phormidium, Planktothrix* et *Raphidiopsis,* ou de pinnamine, toxine marine avec une structure chimique très proche de l'anatoxine-a et ou de toute toxine susceptible d'agir sur des RnACh (Aráoz R, Molgó J, Tandeau de Marsac NT (2009) Neurotoxic cyanobacterial toxins. Toxicon 56: 813-828. [19], Molgó J, Girard E, Benoit, E. (2007) Cyclic imines: an insight into this emerging group of bioactive marine toxins. In Phycotoxins: Chemistry and Biochemistry (Botana, L. M., ed) pp. 319-335, Blackwell Publishing Ltd, Iowa.[4]).

La présente invention à également pour objet l'utilisation d'un dispositif d'analyse selon l'invention pour la détection et la quantification de ligands de récepteurs nicotiniques de l'acétylcholine.

La présente invention à également pour objet l'utilisation d'un dispositif d'analyse selon l'invention pour le criblage à haut débit de ligands de récepteurs nicotiniques de l'acétylcholine.

La présente invention permet avantageusement d'augmenter l'efficacité du recouvrement de fragments membranaires de Torpille sur des surfaces, par exemple des microplaques et/ou des barrettes de part, par exemple le choix du pH, de la force ionique desdites solutions et/ou le choix des solutions d'incorporation, des composants desdites solutions de recouvrement. En outre la présente invention tire profit du développement de la chimie de surface cherchant à maximiser le taux de fixation de protéines par des microplaques en plastique (Esser P (2010). Principles in Adsorption to Polystyrene. Technical Bulletin: 06a. Thermo Fisher Scientific Inc.[18]). En outre, l'augmentation de la fixation, par exemple sur des plaques Maxisorp™ peut impliquer la chimie des surfaces des dites plaques et la nature macromoléculaire des membranes de Torpille. En effet, les fragments membranaires d'électrocytes de Torpille sont des mélanges de lipides (membranes) et de protéines, dont le RnACh peut représenter jusqu'à 40% de la concentration totale de protéine. En plus, les RnACh sont des protéines fortement glycosylées (7,5%) (Da Costa CJB, Kaiser DEE, Baenziger JE (2005). Role of Glycosylation and Membrane Environment in Nicotinic Acetylcholine Receptor Stability. Biophys J, 88, 1755-1764.) [20], ce qui renforce ainsi l'efficacité de la fixation et du recouvrement de la surface, par exemple de plaques Maxisorp™ de part les interactions non-covalentes et la formation de pont d'hydrogène entre les membranes d'électrocyte riche en RnACh et la surface.

Avantageusement, dans la présente invention, la fixation de membranes de cellules électrocytes riches en RnACh permet la détection de quantités faibles, par exemple de l'ordre du nanogramme voir du picogramme, de substances, par exemple de neurotoxines telles qu'indiquées précédemment.

La présente invention a également pour objet l'utilisation d'un dispositif d'analyse selon l'invention pour la purification et caractérisation physico-chimique de ligands de récepteurs nicotiniques de l'acétylcholine.

Avantageusement, le présent dispositif peut également être utilisé afin de purifier et identifier des ligands des récepteurs nicotiniques de l'acétylcholine (RnACh).

Avantageusement, la forte adhésion des fragments membranaires d'électrocytes de Torpille sur la surface des plaques telles que définies ci-dessus, par exemple les plaques de type Maxisorp™ (marque de commerce), ou de plaques à haute adhérence pour des glycoprotéines et membranes à forte concentration de protéine, permet de fixer et piéger au moins un ou plusieurs ligands interagissant avec les RnACh de Torpille.

La récupération du ou des ligands fixés peut être réalisée par exemple, après lavage du dispositif selon l'invention avec un tampon de lavage, par exemple du tampon salin Tris (TBS) contenant 0,1% Tween 20, via une étape d'élution avec une solution d'élution, par exemple du méthanol dans le cas de molécules lipophiles tels que les imines cyclisées ou en employant d'autres tampons à force ionique, pH et concentration en détergent déterminés par l'homme du métier qui puissent libérer les ligands du RnACh de Torpille.

Une fois les ligands élués, leur nature chimique peut être déterminée rapidement, par exemple dans un temps d'environ 5 minutes, par exemple à l'aide de la spectrométrie de masse, de type MALDI-TOF-MS et/ou tout procédé connu de l'homme du métier.

Avantageusement, selon l'invention les fragments membranaires. d'électrocyte de Torpille n'ont pas besoin d'être chimiquement modifiées pour se fixer fortement sur la surface des plaques. La présente invention permet donc avantageusement la fixation des fragments membranaires sans modification structurelle préalable et permet avantageusement de ne pas dénaturer, ni altérer les propriétés biologiques desdits fragments membranaires.

D'autres avantages pourront encore apparaître à l'homme du métier à la lecture des exemples ci-dessous, illustrés par les figures annexées, donnés à titre illustratif.

**Brève description des figures**

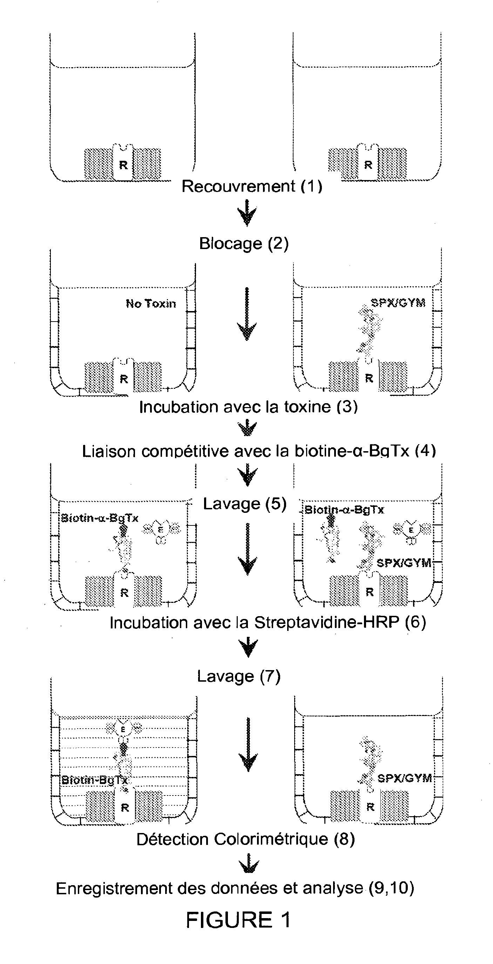

- La figure 1 représente un schéma de l'essai de déplacement de la liaison ligand-récepteur sur microplaque de type ELISA. Le schéma représente les différentes étapes du procédé : (1) recouvrement des puits de microplaque avec des membranes d'électrocytes de Torpille riches en récepteurs nicotiniques de l'acétylcholine (RnACh), (2) blocage des surfaces plastique non liées, (3) incubation des membranes de Torpille immobilisées sur la paroi des puits avec une solution test (soit de la toxine standard ou soit de l'extrait des fruits de mer) (4) Déplacement compétitive du ligand lié au récepteur par de la Biotine-$\alpha$-bungarotoxine (Biotine-$\alpha$-BgTx). (5) lavage. (6) Incubation avec la streptavidine couplée à la peroxydase de raifort (HRP-streptavidine), (7) lavage, (8) détection colorimétrique du complexe HRP-streptavidine - Biotine-$\alpha$-BgTx - RnACh. 9) lecture de la plaque ELISA et analyse des données. Abréviations : R = RnACh, SPX = 13-desméthyl spirolide C/ 13-19 didesméthyl spitolide C, GYM = gymnodimine A, Biotine-$\alpha$-BgTx = biotine-$\alpha$-bungarotoxine, E = HRP-streptavidine.

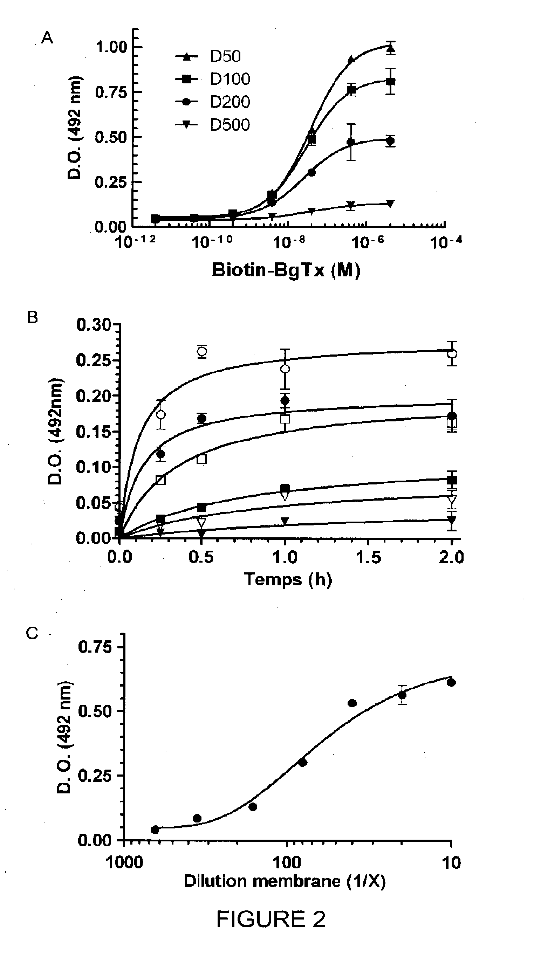

- La figure 2 représente des courbes de standardisation de l'essai de déplacement de la liaison ligand-récepteur sur microplaque de type ELISA.
- La figure 2A représente des courbes de saturation de la liaison entre de la Biotine-$\alpha$-BgTx et les RnACh d'électrocytes de Torpille en employant des solutions 500-fois diluées de membrane (-▲-), 200-fois diluées de membrane (-●-), 100-fois diluées de membrane (-■-) et 50-fois diluées de membrane (-♦-). Chaque dilution a été réalisée dans du tampon salin Tris (TBS). Les membranes ont été incubées pendant une nuit avec différentes concentrations de Biotine-$\alpha$-BgTx ($5 \times 10^{-12}$ M to $5 \times 10^{-6}$ M) afin de déterminer la constante d'affinité de la Biotine-$\alpha$-BgTx pour le RnACh de Torpille.
- La figure 2B est un diagramme représentant la détermination de la concentration de traceur (biotine-$\alpha$-BgTx) pour la méthode. Les puits de microplaques ayant été recouverts avec une solution de membranes d'électrocytes de Torpille diluées 200 fois dans du tampon TBS ont été incubé avec différentes concentrations de biotine-$\alpha$-BgTx: $2 \times 10^{-9}$ M (-▼-), $6 \times 10^{-9}$ M (-▽-), $1 \times 10^{-8}$ M (-■-), $4 \times 10^{-8}$ M (-□-), $8 \times 10^{-8}$ M (-●-) et $2 \times 10^{-7}$ M (-○-) pendant 0, 0,5, 1, 1,5 et 2 h. L'abscisse représente le temps d'incubation en heures (h) et l'ordonnée la densité optique mesurée à une longueur d'onde de 492nm ($DO_{492nm}$).
- La figure 2C est un diagramme représentant la détermination de la dilution de membranes d'électrocyte de Torpille pour l'essai de déplacement de la liaison ligand-récepteur sur microplaque de type ELISA. Différentes dilutions de membrane d'électrocyte de Torpille (10 à 5000 fois diluées) ont été incubées pendant la nuit avec $8 \times 10^{-8}$ M de biotine-$\alpha$-BgTx. L'abscisse représente la dilution des membranes (1/X) et l'ordonnée la densité optique mesurée à une longueur d'onde de 492nm ($DO_{492nm}$). Toutes les expériences ont été réalisées au moins deux fois. Chaque point de la courbe représente la valeur moyenne $\pm$ écart moyen (n = 3).

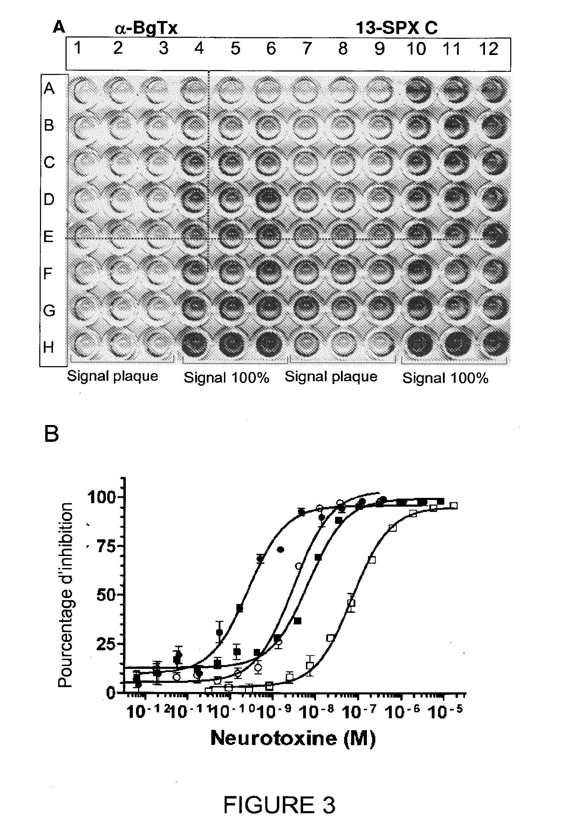

- La figure 3 représente l'inhibition compétitive de la liaison biotine-$\alpha$-BgTx avec le RnACh de Torpille par de l'$\alpha$-bungarotoxine et des phycotoxines portant des imines cyclisées.
- La figure 3A est une photographie d'une microplaque montrant l'inhibition compétitive dose dépendante de la liaison biotine-$\alpha$-BgTx avec des RnACh de Torpille par de la $\alpha$-BgTx standard et par la phycotoxine 13-desméthyl spirolide C. Chaque dose a été testée en triplicate. Sur la partie gauche est représentée la concentration de $\alpha$-BgTx / (puits): $3,3 \times 10^{-6}$ (A1-A3), $1,1 \times 10^{-6}$ (B1-B3), $3,7 \times 10^{-7}$ (C1-C3), $1,2 \times 10^{-7}$ (D1-D3), $4.1 \times 10^{-8}$ (E1-E3), $1,4 \times 10^{-8}$ (F1-F3), $4,6 \times 10^{-9}$ (G1-G3), $1,5 \times 10^{-9}$ (A4-A6), $5,1 \times 10^{-10}$ (B4-B6), $1,7 \times 10^{-10}$ (C4-C6), $5,7 \times 10^{-11}$ (D4-D6), $1,9 \times 10^{-11}$ (E4-E6), $6,2 \times 10^{-12}$ (F4-F6) et $2,09 \times 10^{-12}$ M $\alpha$-BgTx. (G4-G6). Sur la partie droite: concentration de 13-desméthyl SPX C / (puits) : $3,3 \times 10^{-7}$ (A7-A9), $1,1 \times 10^{-7}$ (B7-B9), $3,7 \times 10^{-8}$ (C7-C9), $1,2 \times 10^{-8}$ (D7-D9), $4,1 \times 10^{-9}$ (E7-E9), $1,4 \times 10^{-9}$ (F7-F9), $4,6 \times 10^{-10}$ (G7-G9), $1,5 \times 10^{-10}$ (A10-A12), $5,1 \times 10^{-11}$ (B10-B12), $1,7 \times 10^{-11}$ (C10-C12), $5,7 \times 10^{-12}$ (D10-D12), $1,9 \times 10^{-12}$ (E10-E12), $6,2 \times 10^{-13}$ (F10-F12) et $2,1 \times 10^{-13}$ M 13-desméthyl SPX C (G10-G12). La α-BgTx et 13-desméthyl SPX C inhibent de façon compétitive et dose dépendante, mais dans une ampleur différente, la liaison de la biotine-á-BgTx au RnACh des membranes de Torpille immobilisées. Le signal plaque est le contrôle négatif : puits sans recouvrement par les membranes. 100% signal est le contrôle positif: il s'agit de puits dans lesquels des membranes de Torpille ont été mises en absence de toxines ou d'échantillons d'extrait.

- La figure 3B est un diagramme représentant l'inhibition dose-dépendante de la liaison biotine-α-BgTx avec des RnACh de Torpille immobilisées en utilisant l'essai de déplacement de la liaison ligand-récepteur sur microplaque de type ELISA par l'α-BgTx (-●-), le 13-19-didesméthyl spirolide C (-○-), le 13-desméthyl spirolide C (-■-) et par la gymnodimine A (-□-). Des expériences indépendantes ont été réalisées au moins trois fois. Chaque point représente la valeur moyenne ± écart moyen (n = 3). L'abscisse représente la concentration de toxine (M) et l'ordonnée le pourcentage d'inhibition.

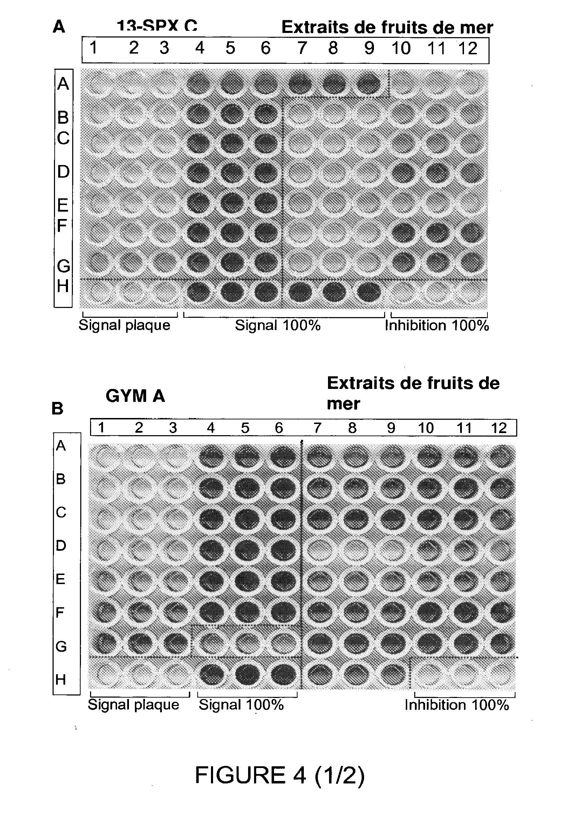

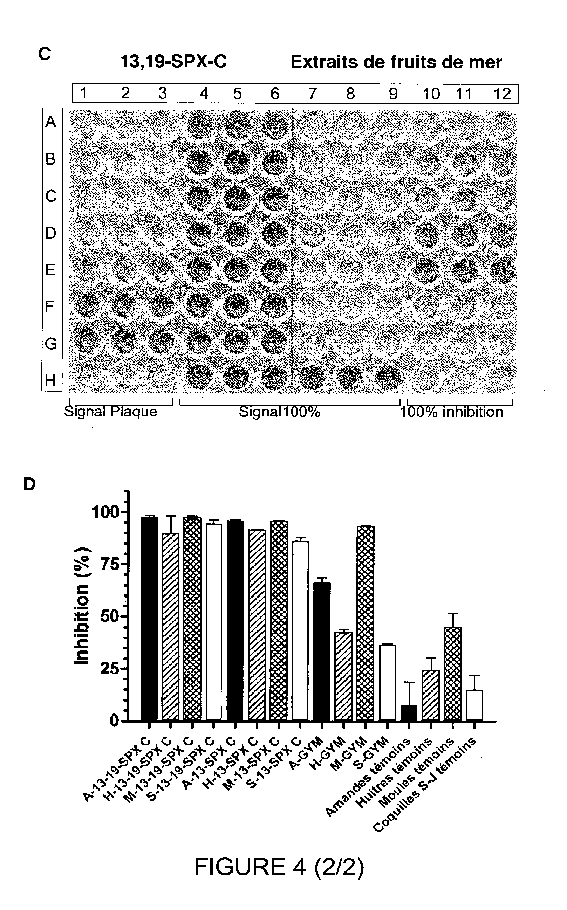

- La figure 4 représente l'inhibition compétitive de la liaison biotine-α-BgTx avec le RnACh de Torpille par des phycotoxines portant des imines cyclisées et des extraits de fruits de mer témoins et contaminés.

- La figure 4A est une photographie d'une microplaque montrant l'inhibition compétitive dose dépendante de la liaison biotine-α-BgTx avec des RnACh de Torpille par du 13-desméthyl SPX C et des extraits de fruit de mer contaminés avec ladite toxine et des extraits témoins. Chaque dose a été testée en triplicate. Sur la partie gauche: concentration de 13-desméthyl spirolide (puits) : $1,7 \times 10^{-5}$: (A1-A3), $5,6 \times 10^{-6}$: (B1-B3), $1,9 \times 10^{-6}$: (C1-C3), $6,2 \times 10^{-7}$: (D1-D3), $2,1 \times 10^{-7}$: (E1-E3), $6,9 \times 10^{-8}$: (F1-F3), $2,3 \times 10^{-8}$: (G1-G3), $7,6 \times 10^{-9}$: (A4-A6), $2,5 \times 10^{-9}$: (B4-B6), $8,5 \times 10^{-10}$: (C4-C6), $2,8 \times 10^{-10}$: (D4-D6), $9,4 \times 10^{-11}$: (E4-E6), $3,1 \times 10^{-11}$: (F4-F6) et $1,0 \times 10^{-11}$ M 13-SPX-C: (G4-G6). Sur la partie droite est représentée la concentration de 13-desméthyl spirolide de $3,5 \times 10^{-12}$ M (A7-A9) et les extraits de fruits de mer. Extraits de fruit de mer dopés avec du 13-desméthyl spirolide C-dilution : (puits). Amandes-$D_{200}$: (B7-B9), amandes-$D_{400}$: (C7-C9), huitres-$D_{200}$: (D7-D9), huitres-$D_{400}$: (E7-E9), moules-$D_{200}$: (F7-F9), moules-$D_{400}$: (G7-G9), coquilles Saint-Jacques-$D_{200}$: (A10-A12) et coquilles Saint-Jacques-$D_{400}$: (B10-B12). Extraits témoins de fruit de mer-dilution: (puits). Amandes témoins-$D_{200}$: (C10-C12), huitres témoins-$D_{200}$: (D10-D12), moules témoins-$D_{200}$: (E10-E12), coquilles Saint-Jacques témoins-$D_{200}$: (F10-F12) et coquilles Saint-Jacques témoins-$D_{400}$: (G10-G12). Signal plaque: puits contrôles négatifs incubées sans recouvrement par une membrane (H1-H3); signal 100%: puits contrôles positifs dans lesquels des membranes de Torpilles ont été incubées sans toxines ou sans extrait (H4-H9); 100% inhibition : puits comprenant des membranes de Torpilles incubés avec $1 \times 10^{-5}$ M de α-BgTx (H10-H12).

- La figure 4B est une photographie d'une microplaque montrant l'inhibition compétitive dose dépendante de la liaison biotine-α-BgTx avec des RnACh de Torpille par de la gymnodimine-A, des extraits de fruit de mer contaminés avec ladite toxine et des extraits témoins. Chaque dose a été testée en triplicate. Sur la partie gauche : concentration de gymnodimine-A (puits) : $1,7 \times 10^{-5}$: (A1-A3), $5,6 \times 10^{-6}$: (B1-B3), $1,9 \times 10^{-6}$: (C1-C3), $6,2 \times 10^{-7}$: (D1-D3), $2,1 \times 10^{-7}$: (E1-E3), $6,9 \times 10^{-8}$: (F1-F3), $2,3 \times 10^{-8}$: (G1-G3), $7,6 \times 10^{-9}$: (A4-A6), $2,5 \times 10^{-9}$: (B4-B6), $8,5 \times 10^{-10}$: (C4-C6), $2,8 \times 10^{-10}$: (D4-D6), $9,4 \times 10^{-11}$: (E4-E6) et $3,1 \times 10^{-11}$ M GYM-A: (F4-F6). Extraits d'amandes dopées avec de la GYM A-Dilution: (puits). Amandes-$D_{200}$: (G4-G6). Coté droit: extraits de fruits de mer dopés avec de la GYM A-Dilution: (puits). Amandes-$D_{400}$: (A7-A9), huitres-$D_{200}$: (B7-B9), huitres-$D_{400}$: (C7-C9), moules-$D_{200}$: (D7-D9), moules-$D_{400}$: (E7-E9), coquilles Saint-Jacques-$D_{200}$: (F7-F9) et coquilles Saint-Jacques-$D_{400}$: (G7-G9). Extraits témoins de fruit de mer-dilution: (puits). Amandes témoins-$D_{200}$: (H7-H9), amandes témoins-$D_{400}$: (A10-A12), huitres témoins-$D_{200}$: (B10-B12), huitres témoins-$D_{400}$: (C10-C12), moules témoins-$D_{200}$: (D10-D12), moules témoins-$D_{400}$: (E10-E12), coquilles Saint-Jacques témoins-$D_{200}$: (F10-F12) et coquilles Saint-Jacques témoins-$D_{400}$: (G10-G12). Signal plaque: (H1-H3); signal100%: (H4-H6); 100% inhibition : (H10-H12).

- La figure 4C est une photographie d'une microplaque montrant l'inhibition compétitive dose dépendante de la liaison biotine-α-BgTx avec des RnACh de Torpille par du 13-19 didesméthyl spirolide C, des extraits de fruit de mer contaminés avec ladite toxine et des extraits témoins. Chaque dose a été testée en triplicate. Sur la partie gauche : Concentration 13-19 didesméthyl spirolide C (puits) : $3,3 \times 10^{-7}$ M (A1-A3), $1,1 \times 10^{-7}$ M (B1-B3), $3,7 \times 10^{-8}$ M (C1-C3), $1,2 \times 10^{-8}$ M (D1-D3), $4,1 \times 10^{-9}$ M (E1-E3), $1,4 \times 10^{-9}$ M (F1-F3), $4,6 \times 10^{-10}$ M (G1-G3), $1,5 \times 10^{-10}$ M (A4-A6), $5,1 \times 10^{-11}$ M (B4-B6), $1,7 \times 10^{-11}$ M (C4-C6), $5,7 \times 10^{-12}$ M (D4-D6), $1,9 \times 10^{-12}$ M (E4-E6), $6,2 \times 10^{-13}$ M (F4-F6) et $2,1 \times 10^{-13}$ M 13-19-SPX C (G4-G6). Coté droit: Extraits de fruits de mer dopés avec du 13-19 didesméthyl spirolide C-Dilution: (puits). Amandes-$D_{200}$: (A7-A9), amandes-$D_{400}$: (B7-B9), moules-$D_{200}$: (C7-C9), moules-$D_{400}$: (D7-D9), huitres-$D_{200}$: (E7-E9), huitres-$D_{400}$: (F7-F9), coquilles Saint Jacques-$D_{200}$: (G7-G9) et coquilles Saint Jacques-$D_{400}$: (A10-A12). Extraits témoins de fruit de mer-dilution: (puits). Amandes témoins-$D_{200}$: (B10-B12), huitres témoins-$D_{200}$: (C10-C12), moules témoins-$D_{200}$: (D10-D12), coquilles Saint-Jacques témoins-$D_{200}$: (E10-E12). Extraits des cyanobactéries-Dilution: (puits). PCC 6506-$D_{100}$ : (F10-F12) et PCC 10111-$D_{100}$ : (G10-G12). Signal plaque: (H1-H3); signal 100%: (H4-H9); 100% inhibition: (H10-H12).

- La figure 4D est un diagramme en bâton représentant l'effet matrice. Des homogénats de fruits de mer (amandes = A; huitres = H; moules = M et coquilles St Jacques = S) ont été dopés avec du 13-19-didesméthyl spirolide C, 13-desméthyl spirolide C et gymnodimine A. Les résultats ont été obtenus avec l'essai de déplacement de la liaison ligand-récepteur sur microplaque de type ELISA. Les bâtons représentent la valeur moyenne ± erreur type sur la moyenne (n = 3 pour les échantillons dopés et n=9 pour les échantillons de fruit de mer témoins). L'abscisse représente les échantillons testés et l'ordonnée le pourcentage d'inhibition. 13-19-SPX C = 13-19-didesméthyl spirolide C, 13-SPX C = 13-desméthyl spirolide C, GYM = gymnodimine A.

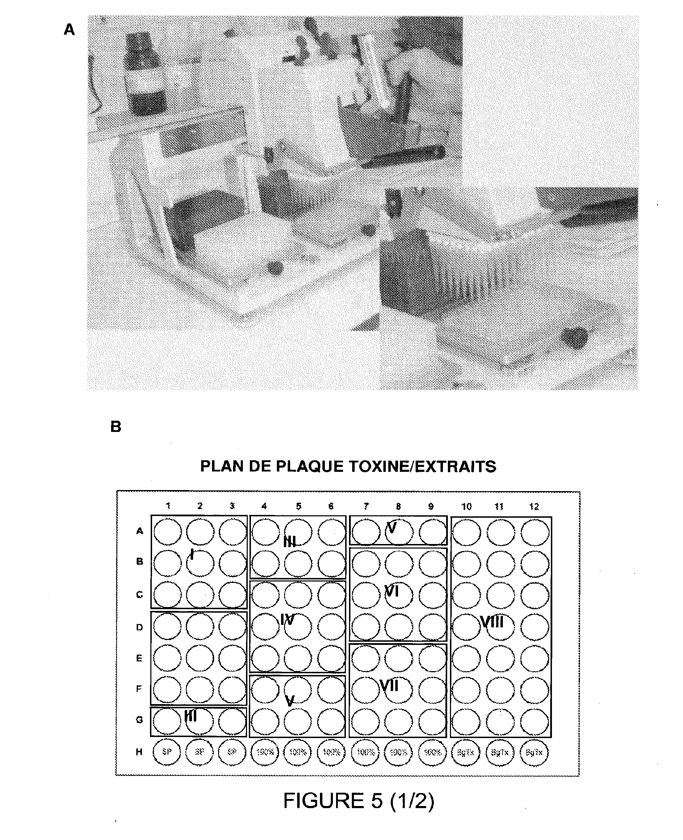

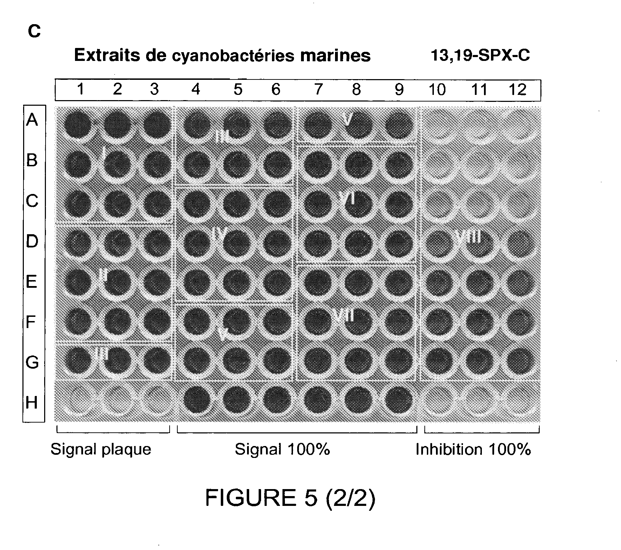

- La figure 5 représente le criblage des toxines/ligands compétitifs des RnACh à partir des extraits de cyanobactéries marines en employant l'essai de déplacement de la liaison ligand-récepteur sur microplaque de type ELISA avec le pipeteur manuel à 96 cônes.
- La figure 5A est une photographie du pipeteur manuel à 96 cônes montrant les zones de charge et pipetage.
- La figure 5B représente le plan de plaque pour le criblage de toxines/ligands compétitifs des RnACh à partir des extraits de cyanobactéries marines I à VII, en employant comme control positif le 13-19-SPX C (VIII). SP (signal de plaque), 100% (Signal 100%). BgTx (1 x $10^{-6}$ M $\alpha$-bungarotoxine : 100% inhibition).
- La figure 5C est une photographie d'une microplaque montrant le criblage de toxines/ligands compétitifs des RnACh à partir des extraits de cyanobactéries marines I à VII testés à trois dilutions : $D_{10}$: première rangé, $D_{100}$: deuxième rangé et $D_{1000}$ troisième rangé. Le rectangle VIII montre sur la même plaque l'inhibition compétitive dose dépendante de la liaison biotine-$\alpha$-BgTx avec des RnACh de Torpille par du 13-19 didesméthyl spirolide C : première rangé : 5 X $10^{-7}$ M, deuxième rangé : 5 X $10^{-8}$ M, troisième rangé : 5 X $10^{-9}$ M, quatrième rangé : 5 X $10^{-10}$ M, cinquième rangé : 5 X $10^{-11}$ M, sixième rangé : 5 X $10^{-12}$ M et septième rangé : 5 X $10^{-13}$ M. Signal plaque: (H1-H3); signal 100%: (H4-H9); 100% inhibition : (H10-H12).

## EXEMPLES

**Exemple 1 : Procédé de détection de l'interaction entre les récepteurs nicotiniques de l'acétylcholine (RnACh) et des toxines**

### Produits utilisées

Dans l'exemple ci-dessous, les produits, dispositifs utilisés étaient les suivants :

Les plaques à fonds plats ELISA Maxisorp ont été achetées à la société NUNC (Kamstrupvej, Denmark).
Le tampon de recouvrement était le suivant: tampon tris salin (TBS) (150 mM chlorure de sodium, 50 mM Tris-HCl, pH 7.5)
Le tampon de lavage : du TBS contenant 0,1% Tween 20.
Le tampon de blocage: du TBS contenant 0,5% albumine de sérum bovin (BSA)
Une solution stock 10 fois concentrée de tampon TBS à pH 7 a été préparé et a été autoclavée et conservée à température ambiante, à savoir à 25°C pour préparer lesdits tampons
La $\alpha$-bungarotoxine biotinylée (Biotin-$\alpha$-BgTx) a été acheté à la société Molecular Probes (Eugene, OR, USA)

Le mélange d'inhibiteurs de protéase complet (« Protease inhibitors mix Complete ») a été acheté à la société Roche Diagnostics GmbH, Mannheim, Germany). L'albumine de sérum bovin (BSA) provenait de la société Sigma-Aldrich (St. Louis, MO, USA). La streptavidine couplée à la péroxidase a été achetée à la société Sigma-Aldrich. Les tablettes o-Phénylènediamine dihydrochloride ont été achetées à la société DAKO (Glostrup, Dennmark). La $\alpha$-bunga-rotoxine a été achetée à la société Sigma-Aldrich. Le 13-Desméthyl spirolide C a été acheté à la société NRC-CNRC (institut de biosciences marine, conseil national de la recherche, Halifax, NS, Canada). La Gymnodimine A a été achetée à la société NRC-CNRC Le 13-19-didesméthyl spirolide C a été acheté à la société CIFGA (Lugo, Spain). L'anatoxin-a provenait de la société TOCRIS (Ellisville, MO, USA)

### Protocole :

La figure 1 représente les différentes étapes de mise en oeuvre du procédé.

Recouvrement avec les membranes (« Coating »)

Une plaque Elisa à fonds plats de 96 puits NUNC Maxisorp™ a été recouverte avec 100 $\mu$l par puits de membranes d'électrocyte de Torpille dont la concentration totale de protéine était de 13.5 $\mu$g/ml dans du tampon de recouvrement TBS (150 mM NaCl, 50 mM Tris-HCl, pH 7,5 soit 1,35 $\mu$g de protéine par puits). Les plaques ont été fermées et incubées à température ambiante (25°C) pendant 2 heures ou toute la nuit à 4°C.

La solution stock de membranes a été dilué 200 fois dans du tampon TBS avant le recouvrement des plaques. Le recouvrement a été effectué avec un pipeteur électronique multicanaux eLINE (Biohit, Helsinki, Finlande) qui a permis d'accélérer le chargement des membranes. Trois puits ont été laissés sans recouvrement avec des membranes pour leur utilisation comme puits contrôle négatif (signal plaque).

Blocage

L'excès de membrane a été éliminé. Le tampon résiduel sur la plaque a été éliminé avec du papier absorbent.

Sans lavage, les puits ont été remplis avec 250 $\mu$l de tampon de blocage (TBS, 0,5 % BSA) par puits. La plaque a été fermée et incubée pendant une heure à température ambiante, à savoir 25°C.

Les plaques ont été stockées à 4°C pour utilisation ultérieure. Des expériences en parallèles ont été réalisées avec des plaques recouvertes six mois avant et avec des plaques recouvertes le jour précédent : aucune différence significative n'a été observé entre les différentes plaques pour les courbes dose réponse de liaison du ligand (résultats non représentés).

Incubation avec les toxines/extraits

Le tampon de blocage a été éliminé et le liquide résiduel sur le haut de la plaque a été absorbé avec un papier absorbant. Sans lavage, les plaques ont été chargées soit avec a) 100 $\mu$l de toxines standard préparées dans du tampon de blocage ou avec b) 100 $\mu$l de tampon de blocage contenant jusqu'à 10% d'extraits (volume/volume). Chaque concentration de toxine ou d'échantillon a été testée 3 fois. La plaque a été fermée et incubée pendant trois heures sous agitation constante à température ambiante (25°C), ou sur la nuit à 4°C pour une sensibilité maximale.

Six puits recouvert avec des membranes de Torpille ont été laissés libres de toxine ou d'extrait pour les utiliser comme contrôle négatifs (Signal 100%). En plus, trois puits recouvert avec des membranes de Torpille ont été laissés libres de toxine ou d'extrait pour les utiliser comme contrôle d'inhibition non-spécifique. Lesdits puits sont incubés avec de 1 $\mu$M $\alpha$-bungarotoxine (100% inhibition).

La concentration de méthanol a été gardée inférieur à 1% afin d'éviter toute interférence avec la liaison du ligand au RnACh.

Déplacement de la Biotine-BgTx:

Sans enlever les toxines et/ou les extraits, 50 $\mu$l de Biotine-$\alpha$-BgTx (8 x $10^{-8}$ M, concentration finale) a été ajouté dans chaque puits. La plaque a été fermée et l'ensemble a été incubé pendant 30 minutes sous agitation constante à température ambiante (25°C).

Les toxines, extraits ou Biotine-$\alpha$-BgTx non liés ont été éliminés et les puits ont été lavés trois fois avec 250 $\mu$l de tampon de lavage (TBS, 0,1% Tween 20). Le tampon résiduel a été absorbé avec du papier absorbant.

Réaction Straptavidine-Biotine

Une quantité de 100 $\mu$l de streptavidine couplée à la peroxydase (diluée 5000 fois, ~220 ng/nl protéine, SIGMA) a été ajoutée à chacun des puits avec un pipeteur électronique multicanaux eLINE (marque déposée) (Biohit). La plaque a été fermée et incubée pendant 30 minutes à température ambiante (25°C) sous agitation constante, c'est-à-dire une agitation d'environ 50 tours par minute.

La pipeteur électronique multicanaux permet de minimiser les différences dans les temps d'exposition à la réaction entre la partie biotine du traceur et la partie sptreptavidine de l'enzyme. Cependant, due au fait de la grande affinité entre la biotine et la streptavidine ($K_d$ = $10^{-14}$ M), une réaction de 30 minutes assure un effet négligeable lié à la différence du temps d'exposition pour chaque rangé de puits. Le temps de réaction entre la Biotine-$\alpha$-BgTx et la Streptavidine-péroxidase à été déterminé expérimentalement, à savoir des membranes d'electrocyte de Torpille immobilisées sur des puits de la microplaque Maxisorp™ ont été incubées avec 8 x $10^{-8}$ M biotin-$\alpha$-BgTx pendant 30 minutes à 25°C. Après lavage, 100 $\mu$l de streptavidine couplée à la peroxydase (diluée 5000-fois) ont été ajouté à chaque puits. Le temps d'incubation a été de 0, 1, 2,5, 5, 10, 15 et 30 min. Les puits ont été lavés et l'activité peroxidase détecté comme décris ci-dessous.

Détection colorimétrique

L'excès de Streptavidine-péroxidase a été éliminé et les puits ont été lavés trois fois avec 250 $\mu$l de tampon de lavage (TBS, 0,1% Tween 20). Le tampon résiduel sur la plaque a été éliminé avec du papier absorbant. Une quantité de 100 $\mu$l de substrat de peroxydase OPD (DAKO, Glostrup, Dennmark) a été ajouté à chaque puits avec un pipeteur électronique multicanaux. Lorsqu'une couleur convenable s'est développée, c'est-à-dire après environ 5 minutes d'incubation, la réaction enzymatique a été stoppée en ajoutant 100 $\mu$l de 0,5 M $H_2SO_4$ à chaque puits avec un pipeteur électronique multicanaux.

L'utilisation d'un pipeteur électronique multicanaux permet d'éviter les variations dans le développement de la couleur dues à la différence de temps d'exposition. La solution de stoppage a été ajoutée dans le même ordre que la solution de substrat de la péroxydase (OPD) a été ajoutée afin de minimiser les différences due au temps d'incubation.

Le substrat de la peroxydase (OPD) a été préparé selon les recommandations du fournisseur. Quatre tablettes OPD ont été dissoutes dans 12 ml d'eau déionisée à température ambiante (25°C), suite à quoi après 5 μl de 30% de peroxyde d'hydrogène a été ajoutés.

La plaque a été enregistrée avec un lecteur ELISA (Genios Pro, Tecan), à une absorbance de 492 nm (figure 1).

Le pourcentage d'inhibition a été calculé selon la formule suivante :

$$\text{Inhibition \%} = [100 \times (\text{signal 100\%} - \text{signal de l'échantillon}) / (\text{signal 100\%} - (\text{inhibition 100\%} - \text{signal plaque})]$$

Le signal 100% a été obtenu dans des puits dans lesquels des membranes de Torpille ont été appliquées et incubées sans toxines/extraits; le signal plaque («plate signal ») a été obtenu des puits dans lesquels le recouvrement avec les membranes de Torpille a été omis. Des expériences indépendantes ont été réalisées au moins deux fois avec des triplicates.

Le pourcentage d'inhibition a été également calculé selon la formule suivante :

$$\text{Inhibition \%} = [100 \times (\text{signal 100\%} - \text{signal de l'échantillon}) / (\text{signal 100\%} - \text{inhibition 100\%})]$$

Le signal 100% a été obtenu dans des puits dans lesquels des membranes de Torpille ont été appliquées et incubées sans toxines/extraits; le signal inhibition 100% («inhibition 100%») a été obtenu des puits dans lesquels les membranes de Torpille ont été incubées avec de l'α-bungarotoxine (1 ou 10 μM). Des expériences indépendantes ont été réalisées au moins deux fois avec des triplicates.

Les résultats ont montrées que des antagonistes des RnACh, a savoir: l'α-bungarotoxine, le 13-desméthyl spirolide C, le 13,19 didiesmethyl spirolide C, la gymnodimine, le d-Tubocurarine (curare) et des agonistes des RnACh, à savoir : l'anatoxina-a et l'homoanatoxine-a, inhibent l'interaction entre de l'α-BgTx et le RnACh de Torpille de façon dépendante à leur concentration avec des affinités différentes (figures 3A, 3B, 4A, 4B et 4C).

Ce système est aussi utilisable avec de la streptavidine couplé à la phosphatase alcaline en plus de la streptavidine couplé à la peroxydase de raifort, et, comme révélateurs des enzymes des substrats soit produisant une réaction colorimétrique (OPD, Nitro blue tetrazolium chloride), soit une substance décelable par chimioluminescence (ECL +) (Aráoz R, Herdman M, Rippka R, Ledreux A, Molgó J, Changeux JP, Tandeau de Marsac N, Nghiêm HO. (2008). A non-radioactive ligand-binding assay for detection of cyanobacterial anatoxins using Torpedo electrocyte membranes. Toxicon 52:163-174.**[14]**). La liste de révélateurs n'est pas limitative.

Tableau 1. Sensibilité de l'essai non radioactif de déplacement de la liaison ligand-récepteur sur microplaque de type ELISA pour la détection de α-bungarotoxine (α-BgTx), 13 desméthyl spirolide C (13-SPX C), 13-19 didesméthyl spirolide C (13-19-SPX C), gymnodimine A (GYM A) et de l'anatoxine-a (ANTX)

| | α-BgTx | 13-SPX-C | 13,19-SPX-C | GYM-A | ANTX |
|---|---|---|---|---|---|
| $IC_{50}$ (95% CI) | 0,27 (0,18-0,41) | 6,84 (5,07-9,22) | 3,10 (1,98-4,85) | 74,23 (62,4-88,3) | 68,81 (35,3-34,2) |
| LOD (nM) | 0,02 | 1 | 0,8 | 2 | 2 |
| LOQ (nM) | 0,20 | 4 | 2,0 | 20 | 30 |
| $IC_{90}$ (nM) | 70 | 60 | 20 | 2000 | 2000 |

L'$IC_{50}$ représente la concentration de neurotoxine à laquelle il y a 50% d'inhibition de la liaison entre la biotine-α-BgTx et les RnACh de Torpille ; le 95% CI est l'intervalle de confiance à 95% de la valeur $IC_{50}$ correspondante ; le LOD est la limite de détection de la méthode, le LOQ, est la limite de quantification de la méthode ; l'$IC_{90}$ représente la concentration de neurotoxine nécessaire à inhiber 90% de la liaison entre la biotine-α-BgTx et les RnACh de Torpille.

L'essai non radioactif de déplacement de la liaison ligand-récepteur sur microplaque de type ELISA est une méthode fonctionnelle basée sur le mécanisme d'action des ligands compétitifs des RnACh. Cette méthode permet de détecter des agonistes et antagonistes compétitifs des RnACh avec une grande sensibilité.

La sensibilité de la méthode utilisant les dispositifs de la présente invention a été comparée avec la Chromatographie Liquide Ultra Performante couplé à la Spectrométrie de Masse (UPLC-MS/MS) pour la détection de 13-SPX C, 13-19-SPX C et GYM A [Aráoz et coll., manuscrit en préparation]. Les valeurs de LOD et LOQ obtenus par UPLC-MS/MS montrent que la sensibilité de la méthode de déplacement non-radioactive utilisant le dispositif de la présente invention est dans le même ordre de grandeur que celle de l'UPLC-MS/MS (Tableau 2).

Tableau 2. Sensibilité de l'UPLC-MS/MS pour la détection de 13-SPX C, 13-19-SPX C et GYM A

|  | 13-SPX-C | 13,19-SPX-C | GYM-A |
|---|---|---|---|
| LOD (nM) | 4,78 | 1,66 | 1,52 |
| LOQ (nM) | 13,1 | 5,55 | 4,17 |

Tel que démontré dans cet exemple, l'utilisation du dispositif obtenu par le procédé de l'invention permet de détecter l'interaction entre les RnAch et des ligands avec une sensibilité aussi grande que les techniques les plus précises de l'état de la technique sans utilisation d'un dispositif complexe et/ou couteux et cela parallèlement sur un grand nombre d'échantillons (96) pour la détection simultanée des plusieurs ligands/agonistes/antagonistes des RnACh à savoir les spirolides, gymnodimines, pinnatoxines, anatoxines, curare, ...(la liste n'est pas limitative).

**Exemple 2 : Procédé de détection du 13 desméthyl spirolide C, du 13,19 didesméthyl spirolide C et de la gymnodimine A à partir des extrais des fruits de mer et de l'anatoxin-a à partir des extraits de cyanobactéries**

Dans cet exemple, les solutions et protocoles suivants ont été utilisés :

- Tampon de recouvrement (« coating »): TBS (150 mM chlorure de sodium, 50 mM Tris-HCl, pH 7,5).

- Dilution des membranes: 200 fois. Un volume de 50 $\mu$l de membrane de Torpille (2.7 mg ml$^{-1}$ de protéine dans 5 mM de glycine) a été dilué dans 10 ml TBS.

- Support: plaques ELISA à fonds plats Maxisorp (NUNC).

- Volume de recouvrement (« coating »): 100 $\mu$l de membranes de Torpille diluées 200 fois

- Température et temps de recouvrement (« coating »): température ambiante (25°C) pendant 2 heures ou 4°C sur la nuit.

- Extraits de fruits de mer (amandes, huitres, moules et coquilles Saint-Jacques) témoins et dopés avec 13 desméthyl spirolide C, 13,19 didesméthyl spirolide C et gymnodimine A

- Extraits de cyanobactéries productrices d'anatoxin-a et homoanatoxin-a

Une plaque ELISA 96 puits à fonds plats Maxisorp (NUNC) a été recouverte avec 100 $\mu$l par puits d'une solution de membranes d'électrocyte de Torpille (13,5 $\mu$g/ml protéine total, soit 1,35 $\mu$g de protéine membranaire par puits) dans du tampon TBS (150 mM NaCl, 50 mM Tris-HCl, pH 7,5) à l'aide d'un pipeteur multicanaux. La plaque a été fermée et incubée à température ambiante (25°C) pendant 2 heures ou à 4°C sur la nuit.

Détection des phycotoxines dans des extraits de fruits de mer

Extraction des toxines lipophiliques à partir de fruits de mer :

La préparation des extraits bruts à partir d'échantillons de fruits de mer a été réalisée comme suit : de la chaire d'amandes, huitres, moules ou coquilles Saint-Jacques (100g) a été homogénéisé dans un broyeur à couteaux à puissance maximale et divisée en 10 aliquotes et gardé à - 80°C.

50 $\mu$l de 13 desméthyl spirolide C standard, 50 $\mu$l de 13,19 didesméthyl spirolide C standard ou 50 $\mu$l de gymnodimine A standard, à une concentration de 10 $\mu$M tout les trois ; ont été ajouté séparément sur 1 g de chaque homogénat de fruit de mer.

Pour extraire ces phycotoxines lipophiliques, 4 ml d'acétone a été ajouté sur chacun d'homogénats dopés. Les mélanges ont été vortéxés pendant 10 secondes et mélangés sur un agitateur mécanique rotatif pendant 15 minutes à température ambiante (25°C). Ensuite, les homogénats ont été centrifugé à 3500 g pendant 15 minutes à 4°C et les surnageants ont été récupérés tandis que les culots ont été re-extrait deux fois avec 4 ml d'acétone comme il vient d'être décrit. Les pools de surnageants ont été évaporés et les résidus ont été resuspendus dans 4 ml d'eau. Sur les extraits aqueux, 4 ml d'hexane ont été ajoutés et laissé pendant 30 min à température ambiant (25°C) pour que la partition chimique ait lieu.

Alternativement, les homogénats ont été centrifugés à 3500 tours par minute pendant 15 minutes à 4°C et les surnageants ont été récupérés tandis que les culots ont été re-extrait deux fois avec 4 ml d'acétone comme il vient d'être décrit. Les pools de surnageants ont été évaporés et les résidus ont été resuspendus dans 4 ml d'eau. Sur les extraits aqueux, 4 ml d'hexane ont été ajoutés et laissé pendant 30 min à température ambiant (25°C) pour que la partition chimique ait lieu.

Les phases aqueuses ont été extraites trois fois avec du chloroforme (1:1,5 v/v). Les couches de chloroforme ont été regroupées et évaporées. Les résidus obtenus ont été resuspendus dans 100 μl de méthanol et conservés à -80°C jusqu'à leur utilisation.

1 g d'homogénat de chaque bivalve (amandes, huitres, moules et coquilles St-Jacques) ont été soumis au même traitement pour servir comme témoins.

Préparation des plaques :

Des dilutions sérielles de toxines standards, c'est-à-dire de 13 desméthyl spirolide C ($1,7 \times 10^{-5}$ - $3,5 \times 10^{-12}$ M), 13,19 didesméthyl spirolide C ($3,3 \times 10^{-7}$ - $2,1 \times 10^{-13}$ M) et gymnodimine A ($1,7 \times 10^{-5}$ - $3,1 \times 10^{-11}$ M), (350 μl) ont été préparés avec du tampon de blocage (TBS, 0,5% BSA). De la même manière, chaque extrait de fruit de mer, témoins ou dopés avec lesdites toxines, ont été dilués 100-fois et 200-fois (350 μl) dans du tampon de blocage (TBS, 0,5% BSA). Chaque dose a été analysée en triplicate (100 μl par puits).

Le 13-desméthyl spirolide C, le 13-19-didesméthyl spirolide C et la gymnodimine A sont distribués dans le commerce dans du méthanol. Les concentrations de méthanol ont été conservées à un niveau inférieur à 1 % pour éviter toute interférence avec la méthode

Préparation des extrais de cyanobactéries

Les d'extraits brut de cyanobactéries productrices d'anatoxin-a ont été préparés comme suit :

Le matériel biologique, c'est-à-dire des filaments de cyanobactéries, ont été récoltés par centrifugation (3000 tours par minute à 15°C pendant 10 minutes à partir d'une culture de cyanobactéries. Le culot de cyanobactéries a été resuspendu dans de l'eau distillé et re-centrifugé dans les mêmes conditions et lyophilisés. Les cellules lyophilisées ont été resuspendus dans 50 mM d'acide acétique (10 mg : 2 ml) et lysées par sonification (5 cycles de 1 minute avec des intervalles de 3 minutes dans la glace) avec un Sonifier Branson 250. L'homogénat obtenu a été agité pendant 4 heures protégé de la lumière et ensuite centrifugé à 10 000 tours par minute pendant 30 minutes à 15 °C. Le surnageant a été collecté puis passé à travers une unité de filtration (0,45 μm de pore) et stocké à - 20°C jusqu'à utilisation.

La figure 4A montre sur la même microplaque, l'inhibition dose-dépendante de la liaison biotin-α-BgTx-RnACh par des dilutions sérielles du 13 desméthyl spirolide C et par des extraits de fruit de mer dopés avec la même toxine et des extraits témoins.

La figure 4B montre l'inhibition de la liaison biotin-α-BgTx-RnACh par des dilutions sérielles de gymnodimine A et des extraits de fruits de mer dopés avec de la gymnodimine A, ainsi que par des extraits de fruit de mer témoins testés à deux concentrations (100-fois et 200-fois dilués).

D'autre part, la figure 4C montre l'inhibition de la liaison biotin-α-BgTx-RnACh par des dilutions sérielles de 13,19 didesméthyl spirolide et des extraits de fruits de mer dopés avec la même toxine, ainsi que par des extraits de fruit de mer témoins. En même temps l'effet des extraits obtenus des cyanobactéries neurotoxiques *d'Oscillatoria,* souche PCC 6506 (productrice d'anatoxine-a) et souche PCC 10111 (productrice d'homoanatoxine-a), sur la liaison biotin-α-BgTx-RnACh sont montrés. Les données d'absorption ($OD_{492nm}$) ont été exprimés en pourcentage d'inhibition en employant la formule suivante : Inhibition % = 100 x [(signal 100% - signal de l'échantillon) / (signal 100% - (inhibition 100% - signal plaque))] ou avec la formule suivante. Inhibition % = [100 x (signal 100% - signal de l'échantillon) / (signal 100% - inhibition 100%)]. A partir des données d'inhibition obtenues, une courbe d'inhibition pour chacune des toxines testées a été construit (fig. 3 B). Le paramètre d'inhibition $IC_{50}$ (inhibition 50 : 50% de RnACh sont occupés par la toxine empêchant la liaison biotin-α-BgTx-RnACh) a été calculé. La limite de détection (LOD) et à la limite de quantification (LOQ) de la méthode ont été aussi calculées (voir tableau 1).

Les résultats obtenus démontrent que la méthode non-radioactive de déplacement de la biotin-α-bungarotoxine est applicable pour la détection et quantification des phycotoxines marines, à savoir par exemple la 13 desméthyl spirolide C, 13,19 didesméthyl spirolide C et gymnodimine A à partir d'échantillons de fruits de mer contaminés (figure 4A, 4B, 4C et 4D) et des neurotoxines de cyanobactéries d'eau douce, a savoir de l'anatoxine-a ou de l'homoanatoxine-a (Tableau 1).

La sensitivité de la méthode dépend de l'affinité de la toxine pour le récepteur. Comme démontré dans cette exemple, le seuil de détection de cette méthode non-radioactive est très bas, par exemple 54 picogrammes de 13,19 didesméthyl spirolide C; 69 picogrammes de 13 desméthyl spirolide C et 101 picogrammes de gymnodimine A.

De plus, la méthode nécessite une très faible quantité de membrane électrocyte, par exemple de 1 à 2 $\mu$g de protéines de membranes d'électrocyte de Torpille par puits dans cet exemple (figure 2A, 2B et 2C). C'est à dire, une consommation très réduite de membranes d'électrocyte de Torpille. En plus, lorsque les membranes d'électrocyte de Torpille sont conservées adéquatement, par exemple des aliquotes de 0,5 ml à -80°C, elles sont stables pendant plusieurs années.

Très important : des microplaques recouvertes « coatés » avec des membranes d'électrocyte de Torpille, lorsqu'elles sont conservées à 4°C dans 200 $\mu$l de tampon de blocage (TBS, 0,5% BSA, pH 7,5), peuvent être utilisées jusqu'à six mois après leur fabrication. Il n'y a pas de différence significative entre des plaques recouvertes (« coatés ») la veille ou des plaques recouvertes (« coatés ») six mois auparavant pour la détection de ligands des RnACh. Cette preuve de principe est très utile pour concevoir la fabrication de plaques préalablement recouvertes « pre-coatés » avec des membranes d'électrocyte de Torpille prêtes à l'emploie.

Les plaques obtenues dans cet exemple sont désignés par *Torpedo*-MicroReceptorPlaque.

Tel que démontré dans cet exemple, l'utilisation du dispositif obtenu par le procédé de l'invention permet de détecter l'interaction entre les RnAch et des ligands compétitifs directement sur des extraits méthanoliques des fruits de mer et des extraits aqueux des filaments de cyanobactéries. En outre les dispositifs de la présente invention à savoir les plaques préalablement recouvertes « pre-coatés » avec des membranes d'électrocyte de Torpille prêtes à l'emploie pourraient être transportées et distribuées par la poste pour leur utilisation dans d'autres pays/continents pour la détection des ligands compétitifs des RnACh.

**Exemple 3 : criblage à haut débit des toxines/ligands compétitifs des récepteurs nicotiniques de l'acétylcholine.**

Les microplaques recouvertes avec des membranes d'électrocyte de Torpille qui sont conservées à 4°C obtenus à l'exemple 1 sont adaptés à des méthodes de haut-débit.

**A/ Utilisation du pipeteur manuel à 96 cônes Liquidator 96TM (Rainin) :**

a) Plaques à 96-tubes à préparer avant de commencer la méthode de détection :

Préalablement, un plan de plaque est préparé tel que représenté dans la figure 5B : Sept extraits de cyanobactéries environnementales testées par triplicate à trois dilutions (10-, 100- et 1000-fois) et des dilutions en série de 13,19 didesméthyl spirolide C allant de 5 X $10^{-7}$ a 5 X $10^{-13}$ M en plus des témoins négatifs (control plaque, 3 puits), témoins 100% signal (6 puits) et 100% inhibition (1 X $10^{-5}$ M BgTx, 3 puits) ont été essayé par la méthode.

La Plaque de la figure 6C montre en rectangles la distribution des sept extraits de cyanobactérie marines et du 13,19 didesméthyl spirolide C. Rectangle I [A1-C3], CYA01 ; Rectangle II [D1-F3], CYA02 ; Rectangles III G1-G3 and [A4-B6], CYA03 ; Rectangle IV [C4-E6], CYA04 ; Rectangles V [F4-G6] and A7-A9, CYA 05 ; Rectangle VI [B7-D9], CYA06 ; Rectangle VII [E7-G9], CYA07 ; Rectangle VIII [A10-G12], 13,19 didesméthyl spirolide C. Selon le plan de plaque, des solutions de toxine où d'extrait dans du tampon de bloquage identique à celui de l'exemple 2 précité sont préparées et distribuées dans des plaques à 96 tubes capables de contenir jusqu'à 1 ml de solution par tube : « PLAQUE TOXINE/EXTRAITS ». Pour chaque toxine standard ou échantillon à tester par triplicate, le volume minimum à préparer est de 350 $\mu$l par tube.

Une deuxième plaque de 96 puits contenant une solution de 2,4 x $10^{-7}$ M de biotine-$\alpha$-BgTx dans du tampon de bloquage (TBS, 0,5 % BSA) « PLAQUE BIOTIN-$\alpha$-BgTx » est préparée. Le volume à préparer est dépendant du nombre de plaques à tester (1 microplaque à 96 puits consomme 50 $\mu$l x 96 = 4,8 ml). Cette solution est fraichement préparée.

Une troisième plaque à 96 tubes « PLAQUE PEROXIDASE » contenant streptavidine couplée à la péroxidase ($D_{5000}$ ~220 ng/nl protéine dans du tampon de blocage) est préparée. Cette solution est fraichement préparée. Le volume à préparer est dépendant du nombre de plaques à tester (1 microplaque à 96 puits consomme 100 $\mu$l x 96 = 9,6 ml).

Une quatrième plaque de préparation à 96 tubes indiquée « PLAQUE OPD » contenant le substrat commercial OPD, au moins 120 $\mu$l par puits, pour développer un dispositif de criblage est préparée. Cette solution est fraichement préparée et doit être protégée de la lumière. Le volume à préparer est dépendant du nombre de plaques à tester (1 microplaque à 96 puits consomme 100 $\mu$l x 96 = 9,6 ml). La solution OPD est préparée selon le protocole du fournisseur : 4 tablettes OPD sont dissoutes dans 12 ml d'eau déionisée, dans laquelle 5 $\mu$l de peroxyde d'hydrogène ($H_2O_2$) à 30% est ajoutée.

50 ml de solution « stop » de la réaction peroxydase (0,5 M $H_2SO_4$) est préparée dans une boite rectangulaire adapté au pipeteur manuel à 96 canaux « PLAQUE STOP ».

b) Procédure

Le dispositif de criblage préalablement recouvert « pre-coatés » obtenu selon le procédé décrit dans l'exemple 1, indiqué *Torpedo*-MicroReceptorPlaque ci après, est sorti du réfrigérateur. La plaque est laissée sur un agitateur à température ambiante (25°C) pendant 30 min. Une fois que le dispositif de criblage est à température, le film adhésif est retiré, le tampon de blocage est éliminé et après avoir séché sa surface, la plaque est placée sur la zone de pipetage.

Incubations avec des toxines/extraits :

Le pipeteur manuel à 96 canaux est chargé avec des cônes neufs et réglé sur 100 µl, et la « PLAQUE TOXINE/EXTRAITS » est placée sur une zone de « charge ».

100 µl de la « PLAQUE TOXINE/EXTRAITS » contenant des standards de toxines, des échantillons, des extraits, des produits de synthèse ou des témoins négatifs et positifs sont prélevés et versés dans la *Torpedo*-Micro-ReceptorPlaque placée dans la zone de pipetage.

Le dispositif de criblage est scellé à l'aide d'un film adhésif (Sealing tape, Costar) et laissé incuber les membranes d'électrocyte de Torpille immobilisées avec les échantillons à tester pendant 3 h avec agitation. De préférence, l'incubation se fait sur la nuit à 4°C.

Lorsque l'incubation est faite à 4°C, la *Torpedo*-MicroReceptorPlaque est placée sur un agitateur à température ambiante (25°C) pendant 30 min avant d'être replacée dans la zone de pipetage du Liquidator 96TM.

Incubation avec le traceur biotin-α-BgTx

Des pointes neuves sont chargées et le pipeteur réglé sur 50 µl et la boîte « PLAQUE BIOTIN-α-BgTx » placée sur la zone de « charge ». 50 µl de chaque tube de la « PLAQUE BIOTIN-α-BgTx » est prélevé et versé directement sur la *Torpedo*-MicroReceptorPlaque. La plaque ainsi obtenue correspond au dispositif de criblage et est incubée pendant 30 min sur un agitateur à température ambiante (25°C) après quoi la solution est éliminée et plaque replacée dans la zone de pipetage.

Lavage

Des pointes neuves sont chargées et 200 µl de solution de lavage (TBS, 0,1% Tween 20) sont prélevés et versés dans les puits de *Torpedo*-MicroReceptorPlaque. La solution de lavage est éliminée et la *Torpedo*-MicroReceptorPlaque replacée dans la zone de pipetage. Cette étape est répétée trois fois.

Incubation avec Streptavidine-HRP/ Lavage

100 µl de chaque tube de la « PLAQUE PEROXIDASE » sont prélevés et versés directement sur le dispositif de criblage. La plaque est incubée pendant 30 min sur un agitateur à température ambiante (25°C). La *Torpedo*-Micro-ReceptorPlaque est lavée comme décrit précédemment et est replacée dans la zone de pipetage.

Développement de la réaction peroxidase & stoppage de la réaction

100 µl de chaque puits de la « PLAQUE OPD » sont prélevés et versés sur le dispositif de criblage. La *Torpedo*-MicroReceptorPlaque est incubée jusqu'à ce qu'une coloration optimale soit obtenue, soit environ 5 minutes et 50 µl de la solution « stop » (0,5 M $H_2SO_4$) est versée à l'aide du pipeteur à 96 pointes.

Lecture & analyse des données

La Torpedo-MicroReceptorPlaque est lue sur le lecteur ELISA (GeniosPro, Tecan) et les résultats analysés.

Les résultats sont montrés dans la figure 5. Les témoins plaque, signal 100% et inhibition non-spécifique 100% montrent le bon fonctionnement et la répétitivité du dispositif d'analyse. Dans les sept extraits de cyanobactéries environnementales d'origine marine qui ont été testés à trois dilutions (10 fois: $D_{10}$ : première rangé, 100 fois $D_{100}$: deuxième rangé et 1000 fois $D_{1000}$ troisième rangé) aucun ligands des RnACh n'a été détecté. Sur la même plaque, le 13,19 didesméthyl spirolide C a été testé; première rangé: 5 X $10^{-7}$ M, deuxième rangé: 5 X $10^{-8}$ M, troisième rangé : 5 X $10^{-9}$ M, quatrième rangé : 5 X $10^{-10}$ M, cinquième rangé : 5 X $10^{-11}$ M, sixième rangé : 5 X $10^{-12}$ M et septième rangé : 5 X

$10^{-13}$ M. L'inhibition de la liaison biotin-$\alpha$-BgTx-RnACh par du 13,19 didesméthyl spirolide C est dose-dépendante et confirme les résultats préalablement obtenus pour cette toxine. L'utilisation du pipeteur à 96-pointes accélère le débit de l'analyse (10 microplaques par jour : 960 analyses possibles). En même temps, l'ajout des solutions contenant soit les toxines/extraits soit le traceur, soit la streptavidine-peroxidase, soit le substrat OPD ou la solution stop, est simultanée permettant un control plus exact du temps de réaction ou incubation.

**B/ Utilisation d'un robot de pipetage :**

Les solutions « SOLUTION TOXINE/EXTRAITS », « SOLUTION BIOTIN-$\alpha$-BgTx », « SOLUTION PEROXIDASE », « SOLUTION OPD » et « SOLUTION STOP » sont préparées comme indiqué précédemment.

Une « plaque modèle » à 96 puits est préparé pour les échantillons de toxines/extraits/ témoins selon plan de plaque à tester. Le robot de pipetage est capable d'automatiser la préparation des dilutions des toxines/échantillons, pour la « plaque modèle ».

Les étapes sont similaires aux étapes précitées.

**Exemple 4 : procédé de fabrication de dispositifs de d'analyse**

Les solutions, membranes et conditions expérimentales sont identiques à celles de l'exemple 2 précité.

Une dilution de la solution stock de membrane, 2,7 mg de protéines par ml, préparée selon le procédé décrit dans l'exemple 1-4 précités a été diluée 200 fois dans du tampon TBS à un pH de 7,5.

100 $\mu$l de la solution de membranes de Torpille diluée 200-fois :13,5 $\mu$g ml$^{-1}$ protéine total, soit 1,35 $\mu$g de protéine membranaire par puits, a été distribuée dans les puits d'une microplaque à fonds plats Maxisorp (marque déposée), NUNC, comme représenté sur les figures 3, 4 et 5.

Un film adhésif a été placé sur ladite plaque et la plaque a été incubé à 4°C pendant la nuit ; soit 12-18 heures.

L'excès de membranes a été éliminé par inversion manuel de la plaque et les bords des puits ont été nettoyés avec un papier absorbant.

Enfin 200 $\mu$l de solution de blocage TBS, 0,5 % BSA a été versée dans chaque puits. Un film adhésif a été déposé sur la plaque dite *Torpedo*-MicroReceptorPlaque et conservée à 4°C jusqu'à son utilisation.

Le dispositif d'analysé ainsi préparé est prêt à l'emploi et notamment prêt pour le transport.

**Exemple 5 : comparaison des procédés de détection connus avec le procédé de l'invention**

Dans cet exemple, les résultats de détection obtenus dans les documents de l'art antérieur suivants Fonfria et coll. [12], Vilariño et coll. [13] et Aráoz et coll. [14] ont été comparés avec ceux obtenus avec le procédé et dispositif de la présente invention (exemples 1 et 2 ci-dessus)

Le tableau 3 ci-dessous représente les différents résultats obtenus que ce soit au niveau de la sensibilité du procédé en fonction de la molécule à détecter.

Tableau 3: comparaison du procédé de l'invention avec les procédés connus

| | Fonfria et coll. | Vilarino Natalia et coll. | Aráoz et coll. | Procédé de l'invention |
|---|---|---|---|---|
| Sensibilité (concentration en nM) | 13,19 SPX C: 63,60 ±3 | 13 SPXC:108,2 ± 11.9<br>GYM A: 391,0 ± 55,3 | ANTX$_{CHIM}$ : 62<br>ANTX$_{VISUEL}$ : 17 | 13,19 SPX C: 3,10 (1,98-4,85)<br>13 SPX C : 6,84 (5,07-9,22)<br>GYM A: 74,23 (62,43-88,28)<br>ANTX : 68,81 (35,3-A34,2) |
| Sensibilité (quantité en $\mu$g de toxine dans le puit) | | 13 SPX C: 85 | | 13,19 SPX C: 0,051<br>13 SPX C : 0,069<br>GYM A: 0,101 |

(suite)

|  | Fonfria et coll. | Vilarino Natalia et coll. | Aráoz et coll. | Procédé de l'invention |
|---|---|---|---|---|
| Limite de détection (nM) |  |  | ANTX$_{CHIM}$ : 37 ANTX$_{VISUEL}$ : 9 | 13,19 SPX-C: 0,8 13 SPX C: 1 GYM A: 2) ANTX : 2 |
| Rang de quantification (nM) | 13,19 SPX C : 30 - 150 | 13SPXC:12,5-125.2 GYM A: 12,5 - 500 |  | 13,19 SPX C: 2 - 20 13 SPX C : 4 - 60 GYM A: 20 - 2000 ANTX : 30 - 2000 |
| Lecture des données | Fluorimètre à polarisation | Fluorimètre à polarisation | Détecteur de chimiluminescence/ Visuel | Lecteur de microplaques |
| Automatisation |  |  |  | OUI |
| Dispositif transportable (microplaques) |  |  |  | OUI (Testé par courrier rapide et par avion) |
| Stabilité du dispositif (microplaques) |  |  |  | 6 mois (Testé) |

Tel que démontré dans cet exemple, l'utilisation du dispositif obtenu par le procédé de l'invention permet de détecter l'interaction entre les RnAch et des ligands avec une sensibilité bien plus grande que celle des procédés et dispositif de l'art antérieur.

En outre, le dispositif de la présente invention à savoir les plaques préalablement recouvertes (« pre-coatés ») avec des membranes d'électrocyte de Torpille prêtes à l'emploie peuvent être transportées et distribuées, par exemple par la poste pour leur utilisation dans d'autres pays/continents pour la détection des ligands compétitifs des RnACh.

Enfin, tel que démontré dans cet exemple, les quantités de fragments membranaires utilisées pour fabriquer le dispositif de l'invention sont très inférieures aux quantités nécessaires dans les procédés de l'état de la technique. Ainsi le procédé de l'invention permet avantageusement de diminuer la quantité de fragments membranaires à utiliser et ainsi diminue, par rapport aux procédés de l'art antérieur le nombre de Torpilles à sacrifier tout en améliorant la sensibilité de détection du dispositif de l'invention par rapport aux dispositifs / procédés connus.

**Exemple 6 : Procédé d'élution des imines cyclisées ayant réagi avec les récepteurs nicotiniques de l'acétylcholine de Torpille immobilisés sur la surface des plaques de type ELISA.**

Produits utilisées:

- Les mêmes produits que dans l'exemple 1.
- Un dispositif d'analyse prêt à l'emploie obtenu dans l'exemple 4 précité indiqué *Torpedo*-MicroReceptorPlaque, c'est à dire une plaque recouverte avec des membranes d'électrocyte de Torpille
- Standards de gymnodimine A, 13 desméthyl spirolide C et 13,19 didesméthyl spirolide C
- Méthanol
- 2,5-dihydroxybenzoic acid (DHB)
- Acétonitrile
- Acide trifluoroacétique

Equipements:

- Lecteur de micoplaques ELISA
- MALDI-TOF (Voyager-DETM STR Workstation, Applied BioSystems, Foster City, CA, USA)

Procédure:

Le dispositif d'analyse prêt à l'emploie à été placé à température ambiante, à savoir à 25°C pendant 30 minutes.

Le tampon de blocage a été éliminé et sans lavage, six puits ont été incubés avec 100 $\mu$l de gymnodimine A, ou avec 100 $\mu$l de 13 desmethyl spirolide C, ou avec 100 $\mu$l de 13,19 didesmethyl spirolide C à une concentration de 1 $\mu$M, préparées dans du tampon de blocage (TBS, 0,5 % BSA, pH 7,5).

Le dispositif a été incubé à 4°C pendant la nuit.

Après incubation du dispositif pendant la nuit à 4°C, la plaque a été placée à température ambiante, à savoir à 25°C pendant 30 min, après quoi les puits sont lavés 5 fois avec 250 $\mu$l de tampon de lavage comprenant du TBS, 0,1% Tween 20, pH 7,5.

L'élution de la gymnodimine A a été réalisé avec 50 $\mu$l de méthanol ajoutés dans chacun des six puits préalablement incubés avec cette toxine. Après 5 min d'incubation à température ambiante (25°C) le méthanol a été récupéré. La même procédure a été appliquée pour éluer du 13 desméthyl spirolide C et du 13,19 didesméthyl spirolide C.

Les éluas ont été ensuite analysés par MALDI-TOF. Pour cela, la préparation de la matrice a été réalisée comme suit: 10 mg d'acide 2,5-dihydroxybenzoïque ont été dissous dans 1 ml d'un mélange d'eau-acétonitrile (1:1) contenant 0,3% (v/v) d'acide trifluoroacétique.

Pour l'analyse par MALDI-TOF, 10 $\mu$l de toxine standard (1 $\mu$M) ont été mélangés avec 10 $\mu$l de matrice préparée comme décrit précédemment à l'aide d'un vortex. Un microlitre de ce mélange a été déposé sur la surface d'une plaque porte-échantillon en acier inoxydable (Perseptive Biosystems, Framingham, MA, USA).

De la même façon, 10 $\mu$l de chaque élua ont été mélangés avec 10 $\mu$l de matrice préparée comme décrit précédemment à l'aide d'un vortex. Un microlitre de ce mélange a été déposé sur la surface d'une plaque porte-échantillon en acier inoxydable. Une fois le solvant évaporé (à savoir 2 min après avoir déposé les échantillons), le porte échantillon a été introduit dans le MALDI-TOF pour l'acquisition de données de masse.

Tableau 4 : Caractérisation physico-chimique des éluas obtenus à partir des puits où les RnACh de Torpille ont été incubés avec des imines cyclisées

| Echantillons | MALDI-TOF | | UPLC/MS/MS ([M+H]$^+$) |
|---|---|---|---|
| | Masse ([M+H]$^+$) | Intensité (coups) | |
| Gymnodimine A (standard) | 508,35 | 22000 | 508,4 |
| Gymnodimine A (élua de 6 puits) | 508,34 | 2000 | |
| 13-SPX C (10 $\mu$M standard) | 692,54 | 16000 | 692,4 |
| 13-SPX C (élua de 6 puits) | 692,53 | 4906,4 | |
| 13,19-SPX C (10 $\mu$M standard) | 678,62 | 20000 | 678,4 |
| 13,19-SPX C (élua de 6 puits) | 678,52 | 1800 | |

Tel que démontré dans cet exemple l'utilisation du dispositif obtenu par le procédé de l'invention permet, par exemple de capturer des imines cyclisées que l'on peut après éluer, par exemple avec du méthanol. L'utilisation du dispositif obtenu par le procédé de l'invention permet donc avantageusement de faciliter l'isolement/ la caractérisation des ligands du RnACh. En particulier, il permet avantageusement de faciliter l'isolement / caractérisation physico-chimique d'un agoniste/antagoniste, par exemple compétitif des RnACh. Ainsi, ce dispositif peut être également avantageusement utilisé dans des programmes de recherche de nouvelles toxines.

Listes des références

1. Directive 2010/63/UE du Parlement européen et du Conseil du 22 septembre 2010 relative à la protection des animaux utilisés à des fins scientifiques. Journal officiel de l'Union européenne. DOUEFR, 20 de Octobre de 2010 (http://eu.vlex.com/source/journal-officiel-union-europeenne-1547/issue/2010/10/20/01).

2. Clôture des Assises de la conchyliculture (15/10/2010). Discours de Bruno Le Maire, Ministre de l'Alimentation, de l'Agriculture et de la Pêche) (http://agriculture.gouv.fr/cloture-des-assises-de-la).

3. Fleming LE, Broad K, Clement A, Dewailly E, Elmir S, Knap A, Pomponi SA, Smith S, Gabriele HS, Walsh P (2006) Oceans and human health: Emerging public health risks in the marine environment. Mar. Pollut. Bull., 53: 545-560.

4. Molgó J, Girard E, Benoit, E. (2007) Cyclic imines: an insight into this emerging group of bioactive marine toxins. In Phycotoxins: Chemistry and Biochemistry (Botana, L. M., ed) pp. 319-335, Blackwell Publishing Ltd, Iowa.

5. Amzil Z, Sibat M, Royer F, Masson N, Abadie E. (2007) Report on the first detection of pectenotoxin-2, spirolide-A and their derivatives in French shellfish. Mar. Drugs 5: 168-179.

6. Bourne Y, Radic Z, Aráoz R, Talley TT, Benoit E, Servent D, Taylor P, Molgó J, Marchot P. (2010) Structural déterminants in phycotoxins and AChBP conferring high affinity binding and nicotinic AChR antagonism. Proc. Natl. Acad. Sci. U. S. A. 107: 6076-6081.

7. Kharrat R, Servent D, Girard E, Ouanounou G, Amar M, Marrouchi R, Benoit E, Molgó J. (2008) The marine phycotoxin gymnodimine targets muscular and neuronal nicotinic acetylcholine receptor subtypes with high affinity. J. Neurochem. 107, 952-963.

8. Aráoz R, Servent D, Molgó J, Iorga BI, Fruchart-Gaillard C, Benoit E, Gu Z, Stivala C, Zakarian A. (2011) Total synthesis of pinnatoxins A and G and revision of the mode of action of pinnatoxin A. J. Am. Chem. Soc. 133, 10499-10511.

9. Sivonen K, Jones G. (1999) Cyanobacterial toxins. In Toxic cyanobacteria in water: a guide to their public health consequences, monitoring and management (Chorus, I., ed) pp. 41-111, Bartram, J. E. & F.N. Spon, London.

10. Gugger M, Lenoir S, Berger C, Ledreux A, Druart JC, Humbert JF, Guette C, Bernard C. (2005) First report in a river in France of the benthic cyanobacterium Phormidium favosum producing anatoxin-a associated with dog neurotoxicosis. Toxicon 45, 919-928.

11. Cadel-Six S. Peyraud-Thomas C, Brient L, Tandeau de Marsac N, Rippka R, Mejean A. (2007) Different genotypes of anatoxin-producing cyanobacteria coexist in the Tarn River, France. Applied and Environmental Microbiology 73, 7605-7614.

12. Fonfría ES, Vilariño N, Molgó J, Aráoz R, Otero P, Espiña B, Louzao MC, Alvarez M, Botana LM (2010) Detection of 13,19-didesmethyl C spirolide by fluorescence polarization using Torpedo electrocyte membranes. Anal. Biochem. 403: 102-107.

13. Vilariño N, Fonfría ES, Molgó J, Aráoz R, Botana LM (2009). Detection of Gymnodimine-A and 13-Desmethyl C Spirolide Phycotoxins by Fluorescence Polarization. Analytical Chemistry, 81: 2708-2714.

14. Aráoz R, Herdman M, Rippka R, Ledreux A, Molgó J, Changeux JP, Tandeau de Marsac N, Nghiêm HO. (2008). A non-radioactive ligand-binding assay for detection of cyanobacterial anatoxins using Torpedo electrocyte membranes. Toxicon 52:163-174.

15. Morel N, Marsal J, Manaranche R, Lazereg S, Mazie JC, Israel M (1985). Large-scale purification of presynaptic plasma membranes from Torpedo marmorata electric organ. J. Cell Bol.,1 01: 1757-1762.

16. Hill JA, Nghiêm H-O & Changeux J-P (1991). Serine-specific phosphorylation of nicotinic receptor associated 43K protein. Biochemistry, 30, 5579-5585.

17. Changeux JP (2010). Allosteric receptors: from electric organ to cognition. Annu Rev Pharmacol Toxicol, 50, 1-38.

18. Esser P (2010). Principles in Adsorption to Polystyrene. Technical Bulletin: 06a. Thermo Fisher Scientific Inc.

19. Aráoz R, Molgó J, Tandeau de Marsac NT (2009) Neurotoxic cyanobacterial toxins. Toxicon 56: 813-828.

20. Da Costa CJB, Kaiser DEE, Baenziger JE (2005). Role of Glycosylation and Membrane Environment in Nicotinic Acetylcholine Receptor Stability. Biophys J, 88, 1755-1764.

21. ROMULO ARAOZ ET AL: "Ligand-binding assays for cyanobacterial neurotoxins targeting cholinergic receptors", ANALYTICAL AND BIOANALYTICAL CHEMISTRY, SPRINGER, BERLIN, DE, vol. 397, no. 5, mars 2010, pages 1695-1704.

**Revendications**

1. Procédé de fabrication d'un dispositif d'analyse comprenant des fragments de membranes de Torpilles immobilisés à sa surface comprenant les étapes de :

   a. Fragmentation de membranes isolées à partir de cellules électrocytes de Torpille,

   b. l'incorporation des fragments membranaires obtenus à l'étape a) dans une solution de pH compris de 6,5 à 10

   c. Fixation desdits fragments membranaires à la surface du dispositif.

2. Procédé selon la revendication 1 dans lequel le procédé comprend avant l'étape c) d'immobilisation de membranes sur le dispositif, une étape b') de dilution des fragments membranaires.

3. Procédé selon la revendication 1 ou 2, dans lequel la surface du dispositif est en plastique.

4. Procédé selon l'une quelconque des revendications 1 à 3 dans lequel la concentration de protéine totale dans ladite solution de pH compris de 6,5 à 10 est comprise entre 5 à 200 $\mu$g/ml.

5. Procédé selon l'une quelconque des revendications 1 à 4 dans lequel la solution pour l'incorporation est une solution choisie parmi un tampon tris salin (TBS) comprenant 150 mM NaCl, 50 mM Tris, pH 7,5, ou un tampon phosphate salin (PBS) comprenant 130 mM chlorure de sodium, 10 mM sodium phosphate, pH 7,0 ou un tampon carbonate/bicarbonate comprenant 150 mM chlorure de sodium, 100 mM sodium carbonate, pH 9,5.

6. Procédé selon l'une quelconque des revendications 1 à 5 dans lequel la Torpille est *Torpedo marmorate* ou *Torpedo californica.*

7. Procédé selon l'une quelconque des revendications 1 à 6, dans lequel les membranes sont des membranes plasmiques de cellules électrocytes.

8. Procédé selon l'une quelconque des revendications précédentes dans lequel la surface est une surface de puits de microplaques et/ou de barrettes.

9. Dispositif d'analyse obtenu par le procédé selon l'une quelconque des revendications 1 à 8.

10. Dispositif selon la revendication 9 dans lequel les fragments membranaires sont fixés à la surface de puits.

11. Dispositif selon la revendication 9 ou 10 dans lequel le dispositif est une plaque ELISA à 96 ou 384 puits, ou une plaque à 4, 8 ou 12 puits, ou des barrettes à 6; 8 ou 12 puits.

12. Utilisation d'un dispositif d'analyse selon l'une quelconque des revendications 9 à 11 pour la détection et la quantification de neurotoxines.

13. Utilisation d'un dispositif d'analyse selon la revendication 12 dans laquelle les neurotoxines agissent sur les récepteurs nicotiniques de l'acétylcholine.

14. Utilisation d'un dispositif d'analyse selon l'une quelconque des revendications 9 à 11 pour la détection et la quantification de ligands de récepteurs nicotiniques de l'acétylcholine.

15. Utilisation d'un dispositif selon l'une quelconque des revendications 9 à 11 pour le criblage à haut débit de ligands de récepteurs nicotiniques de l'acétylcholine.

16. Utilisation d'un dispositif selon l'une quelconque des revendications 9 à 11 pour la purification et caractérisation physico-chimique de ligands de récepteurs nicotiniques de l'acétylcholine.

**Patentansprüche**

1. Verfahren zur Herstellung einer Analysevorrichtung umfassend an ihrer Oberfläche immobilisierte Membranfragmente von Zitterrochen, wobei das Verfahren folgende Schritte umfasst:

    a. Fragmentierung der isolierten Membranen von Elektrozytenzellen von Zitterrochen,
    b. Einbringen der in Schritt a) erhaltenen Membranfragmente in eine Lösung mit einem pH-Wert von 6,5 bis 10.
    c. Fixierung der Membranfragmente an der Oberfläche der Vorrichtung.

2. Verfahren nach Anspruch 1, wobei das Verfahren vor dem Schritt c) der Immobilisierung der Membranen auf der Vorrichtung einen Schritt b') der Verdünnung der Membranfragmente umfasst.

3. Verfahren nach Anspruch 1 oder 2, in welchem die Oberfläche der Vorrichtung aus Kunststoff ist.

4. Verfahren nach einem beliebigen der Ansprüche 1 bis 3, in dem die Gesamtproteinkonzentration in der Lösung mit einem pH-Wert von 6,5 bis 10 und zwischen 5 und 200 µg/ml liegt.

5. Verfahren nach einem beliebigen der Ansprüche 1 bis 4, in dem die für das Einbringen gewählte Lösung eine Lösung ist, die unter einer Trisgepufferten Salzlösung (TBS) umfassend 150 mM NaCl, 50 mM Tris, pH 7,5, oder einer phosphatgepufferten Salzlösung (PBS) umfassend 130 mM Natriumchlorid, 10 mM Natriumphosphat, pH 7,0 oder einem Karbonat/Bikarbonatpuffer umfassend 150 mM Natriumchlorid, 100 mM Natriumkarbonat, pH 9,5 ausgewählt wird.

6. Verfahren nach einem beliebigen der Ansprüche 1 bis 5, in dem der Zitterrochen ein *Torpedo marmorate* oder ein *Torpedo californica* ist.

7. Verfahren nach einem beliebigen der Ansprüche 1 bis 6, in dem die Membranen Plasmamembranen von Elektrozytenzellen sind.

8. Verfahren nach einem beliebigen der vorangehenden Ansprüche, in dem die Oberfläche eine Oberfläche von Mikroplatten-Näpfchen und/oder von Leisten ist.

9. Analysevorrichtung, die durch das Verfahren nach einem beliebigen der Ansprüche 1 bis 8 erhalten wird.

10. Vorrichtung nach Anspruch 9, in der die Membranfragmente auf der Oberfläche von Näpfchen fixiert werden.

11. Vorrichtung nach Anspruch 9 oder 10, in der die Vorrichtung eine ELISA-Platte mit 96 oder 384 Näpfchen ist, oder eine Platte mit 4, 8 oder 12 Näpfchen oder Leisten mit 6, 8 oder 12 Näpfchen.

12. Verwendung einer Analysevorrichtung nach einem beliebigen der Ansprüche 9 bis 11 zum Nachweis und zur Quantifizierung von Neurotoxinen.

13. Verwendung der Analysevorrichtung nach Anspruch 12, in der die Neurotoxine auf die nikotinischen Acetylcholinrezeptoren einwirken.

14. Verwendung einer Analysevorrichtung nach einem beliebigen der Ansprüche 9 bis 11 zum Nachweis und zur Quantifizierung von Liganden von nikotinischen Acetylcholinrezeptoren.

15. Verwendung einer Analysevorrichtung nach einem beliebigen der Ansprüche 9 bis 11 für das Hochdurchsatz-Screening von Liganden von nikotinischen Acetylcholinrezeptoren.

16. Verwendung einer Analysevorrichtung nach einem beliebigen der Ansprüche 9 bis 11 für die Reinigung und die physikalisch-chemische Charakterisierung von Liganden von nikotinischen Acetylcholinrezeptoren.

## Claims

1. A method for manufacturing an analysis device comprising Torpedo membrane fragments immobilized on the surface thereof, comprising the steps of:

   a. fragmenting isolated membranes from Torpedo electrocyte cells,
   b. incorporating the membrane fragments obtained in step a) into a solution with a pH between from 6.5 to 10,
   c. attaching said membrane fragments to the surface of the device.

2. The method according to claim 1, wherein the method comprises, before the step c) of immobilizing membranes on the device, a step b') of diluting the membrane fragments.

3. The method according to claim 1 or 2, wherein the surface of the device is made of plastic.

4. The method according to any one of claims 1 to 3, wherein the total protein concentration in said solution with a pH between from 6.5 to 10 is between from 5 to 200 µg/ml.

5. The method according to any one of claims 1 to 4, wherein the solution for the incorporation is a solution selected from a tris buffered saline (TBS) comprising 150 mM NaCl, 50 mM Tris, pH 7.5, or a phosphate buffered saline (PBS) comprising 130 mM sodium chloride, 10 mM sodium phosphate, pH 7.0, or a carbonate/bicarbonate buffer comprising 150 mM sodium chloride, 100 mM sodium carbonate, pH 9.5.

6. The method according to any one of claims 1 to 5, wherein the Torpedo is Torpedo marmorate or Torpedo californica.

7. The method according to any one of claims 1 to 6, wherein the membranes are plasma membranes of electrocyte cells.

8. The method according to any one of the preceding claims, wherein the surface is a microplate and/or strip well surface.

9. An analysis device obtained by the method according to any one of claims 1 to 8.

10. The device according to claim 9, wherein the membrane fragments are attached to the surface of wells.

11. The device according to claim 9 or 10, wherein the device is a 96-or 384-wells ELISA plate, or a 4-, 8- or 12-wells plate, or 6-, 8- or 12-wells strips.

12. Use of an analysis device according to any one of claims 9 to 11 for detecting and quantifying neurotoxins.

13. Use of an analysis device according to claim 12, wherein the neurotoxins act on nicotinic acetylchloline receptors.

14. Use of an analysis device according to any one of claims 9 to 11 for detecting and quantifying nicotinic acetylcholine receptor ligands.

15. Use of a device according to any one of claims 9 to 11 for the high-throughput screening of nicotinic acetylcholine receptor ligands.

16. Use of a device according to any one of claims 9 to 11 for the purification and physicochemical characterization of nicotinic acetylcholine receptor ligands.

Recouvrement (1)

Blocage (2)

Incubation avec la toxine (3)

Liaison compétitive avec la biotine-α-BgTx (4)

Lavage (5)

Incubation avec la Streptavidine-HRP (6)

Lavage (7)

Détection Colorimétrique (8)

Enregistrement des données et analyse (9,10)

FIGURE 1

FIGURE 2

FIGURE 3

FIGURE 4 (1/2)

FIGURE 4 (2/2)

**PLAN DE PLAQUE TOXINE/EXTRAITS**

FIGURE 5 (1/2)

C

**Extraits de cyanobactéries marines**   13,19-SPX-C

Signal plaque   Signal 100%   Inhibition 100%

FIGURE 5 (2/2)

# EP 2 668 496 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 1376124 A2

### Littérature non-brevet citée dans la description

- **FLEMING LE ; BROAD K ; CLEMENT A ; DEWAILLY E ; ELMIR S ; KNAP A ; POMPONI SA ; SMITH S ; GABRIELE HS ; WALSH P.** Oceans and human health: Emerging public health risks in the marine environment. *Mar. Pollut. Bull.,* 2006, vol. 53, 545-560
- Cyclic imines: an insight into this emerging group of bioactive marine toxins. **MOLGÓ J ; GIRARD E ; BENOIT, E.** Phycotoxins: Chemistry and Biochemistry. Blackwell Publishing Ltd, 2007, 319-335
- **AMZIL Z ; SIBAT M ; ROYER F ; MASSON N ; ABADIE E.** Report on the first détection of pectenotoxin-2, spirolide-A and their derivatives in French shellfish. *Mar. Drugs,* 2007, vol. 5, 168-179
- **BOURNE Y ; RADIC Z ; ARÁOZ R ; TALLEY TT ; BENOIT E ; SERVENT D ; TAYLOR P ; MOLGÓ J ; MARCHOT P.** Structural déterminants in phycotoxins and AChBP conferring high affinity binding and nicotinic AChR antagonism. *Proc. Natl. Acad. Sci. U. S. A.,* 2010, vol. 107, 6076-6081
- **KHARRAT R ; SERVENT D ; GIRARD E ; OUANOUNOU G ; AMAR M ; MARROUCHI R ; BENOIT E ; MOLGÓ J.** The marine phycotoxin gymnodimine targets muscular and neuronal nicotinic acetylcholine receptor subtypes with high affinity. *J. Neurochem,* 2008, vol. 107, 952-963
- **ARÁOZ R ; SERVENT D ; MOLGÓ J ; IORGA BI ; FRUCHART-GAILLARD C ; BENOIT E ; GU Z ; STIVALA C ; ZAKARIAN A.** Total synthesis of pinnatoxins A and G and revision of the mode of action of pinnatoxin A. *J. Am. Chem. Soc.,* 2011, vol. 133, 10499-10511
- Cyanobacterial toxins. **SIVONEN K ; JONES G.** Toxic cyanobacteria in water: a guide to their public health consequences, monitoring and management. 1999, 41-111
- **GUGGER M ; LENOIR S ; BERGER C ; LEDREUX A ; DRUART JC ; HUMBERT JF ; GUETTE C ; BERNARD C.** First report in a river in France of the benthic cyanobacterium Phormidium favosum producing anatoxin-a associated with dog neurotoxicosis. *Toxicon,* 2005, vol. 45, 919-928
- **CADEL-SIX S. PEYRAUD-THOMAS C ; BRIENT L ; TANDEAU DE MARSAC N ; RIPPKA R ; MEJEAN A.** Different genotypes of anatoxin-producing cyanobacteria coexist in the Tarn River, France. *Applied and Environmental Microbiology,* 2007, vol. 73, 7605-7614
- **FONFRIA.** détection of 13,19 didesmethyl spirolide C by fluorescense polarization using torpedo electrocyte membranes. *Analytical Biochemistry,* 2010, vol. 403 (1-2), 102-107
- **VILARIÑO.** detection of gymnodimine-A and 13-desmethyl spirolide C phycotoxins by fluorescense polarization. *Analytical Chemistry,* vol. 81 (7), 2708-2714
- **ARÁOZ.** A non radioactive ligand-binding assay for détection of cyanobacterial anatoxins using torpedo electrocytes membranes. *Toxicon,* vol. 52 (1), 163-174
- Ligand-binding assays for cyanobacterial neurotoxins targeting cholinergic receptors. **ROMULO ARAOZ et al.** ANALYTICAL AND BIOANALYTICAL CHEMISTRY. SPRINGER, Mars 2010, vol. 397, 1695-1704
- **MOREL N ; MARSAL J ; MANARANCHE R ; LAZEREG S ; MAZIE JC ; ISRAEL M.** Large-scale purification of presynaptic plasma membranes from Torpedo marmorata electric organ. *J. Cell Biol.,* 1985, vol. 101, 1757-1762
- **HILL JA ; NGHIÊM H-O ; CHANGEUX J-P.** Serine-specific phosphorylation of nicotinic receptor associated 43K protein. *Biochemistry,* 1991, vol. 30, 5579-5585
- **VILARIÑO N ; FONFRIA ES ; MOLGÓ J ; ARÁOZ R ; BOTANA LM.** Detection of Gymnodimine-A and 13-Desmethyl C Spirolide Phycotoxins by Fluorescence Polarization. *Analytical Chemistry,* 2009, vol. 81, 2708-2714
- **CHANGEUX JP.** Allosteric receptors: from electric organ to cognition. *Annu. Rev. Pharmacol. Toxicol.,* 2010, vol. 50, 1-38
- **ESSER P.** Principles in Adsorption to Polystyrene. Technical Bulletin: 06a. Thermo Fisher Scientific Inc, 2010

- Cyclic imines: an insight into this emerging group of bioactive marine toxins. **MOLGÓ J ; GIRARD E ; BENOIT, E.** Phycotoxins: Chemistry and Biochemistry. Blackwell Publishing Ltd, 2007, 319-335
- **ARÁOZ R ; MOLGÓ J ; TANDEAU DE MARSAC NT.** Neurotoxic cyanobacterial toxins. *Toxicon,* 2009, vol. 56, 813-828
- **DA COSTA CJB ; KAISER DEE ; BAENZIGER JE.** Role of Glycosylation and Membrane Environment in Nicotinic Acetylcholine Receptor Stability. *Biophys J,* 2005, vol. 88, 1755-1764
- **ARÁOZ R ; HERDMAN M ; RIPPKA R ; LEDREUX A ; MOLGÓ J ; CHANGEUX JP ; TANDEAU DE MARSAC N ; NGHIÊM HO.** A non-radioactive ligand-binding assay for detection of cyanobacterial anatoxins using Torpedo electrocyte membranes. *Toxicon,* 2008, vol. 52, 163-174
- Directive 2010/63/UE du Parlement européen et du Conseil du 22 septembre 2010 relative à la protection des animaux utilisés à des fins scientifiques. *Journal officiel de l'Union européenne,* 20 Octobre 2010, http://eu.vlex.com/source/journal-officiel-union-europeenne-1547/issue/2010/10/20/01
- **BRUNO LE MAIRE.** *Clôture des Assises de la conchyliculture,* 15 Octobre 2010, http://agriculture.gouv.fr/cloture-des-assises-de-la
- Cyclic imines: an insight into this emerging group of bioactive marine toxins. **MOLGÓ J ; GIRARD E, BENOIT, E.** Phycotoxins: Chemistry and Biochemistry. Blackwell Publishing Ltd, 2007, 319-335
- **AMZIL Z ; SIBAT M ; ROYER F ; MASSON N ; ABADIE E.** Report on the first detection of pectenotoxin-2, spirolide-A and their derivatives in French shellfish. *Mar. Drugs,* 2005, vol. 5, 168-179
- Cyanobacterial toxins. **SIVONEN K ; JONES G.** Toxic cyanobacteria in water: a guide to their public health consequences, monitoring and management. Bartram, J. E. & F.N. Spon, 1999, 41-111
- **CADEL-SIX S. ; PEYRAUD-THOMAS C ; BRIENT L ; TANDEAU DE MARSAC N ; RIPPKA R ; MEJEAN A.** Different genotypes of anatoxin-producing cyanobacteria coexist in the Tarn River, France. *Applied and Environmental Microbiology,* 2007, vol. 73, 7605-7614
- **FONFRÍA ES ; VILARIÑO N ; MOLGÓ J ; ARÁOZ R ; OTERO P ; ESPIÑA B ; LOUZAO MC ; ALVAREZ M ; BOTANA LM.** Detection of 13,19-didesmethyl C spirolide by fluorescence polarization using Torpedo electrocyte membranes. *Anal. Biochem.,* 2010, vol. 403, 102-107
- **VILARIÑO N ; FONFRÍA ES ; MOLGÓ J ; ARÁOZ R ; BOTANA LM.** Detection of Gymnodimine-A and 13-Desmethyl C Spirolide Phycotoxins by Fluorescence Polarization. *Analytical Chemistry,* 2009, vol. 81, 2708-2714
- **MOREL N ; MARSAL J ; MANARANCHE R ; LAZEREG S ; MAZIE JC ; ISRAEL M.** Large-scale purification of presynaptic plasma membranes from Torpedo marmorata electric organ. *J. Cell Bol.,* 1985, vol. 1 01, 1757-1762
- **CHANGEUX JP.** Allosteric receptors: from electric organ to cognition. *Annu Rev Pharmacol Toxicol,* 2010, vol. 50, 1-38
- **ARÁOZ R ; MOLGÓ J ; TANDEAU DE MARSAC NT.** Neurotoxic cyanobacterial toxins. *Toxicon,* 2009, vol. 56, 813-828